(19) Europäisches Patentamt
European Patent Office
Office européen des brevets

(11) Veröffentlichungsnummer: **0 269 806 B1**

(12) **EUROPÄISCHE PATENTSCHRIFT**

(45) Veröffentlichungstag der Patentschrift: **27.02.91**

(21) Anmeldenummer: **87114161.0**

(22) Anmeldetag: **29.09.87**

(51) Int. Cl.⁵: **C07D 231/14**, C07D 231/16, C07D 403/06, C07D 403/04, C07D 413/04, C07D 403/10, A01N 43/56

# (54) Phenylpyrazolcarbonsäurederivate, ihre Herstellung und Verwendung als Pflanzenwachstumsregulatoren und Safener.

(30) Priorität: **04.10.86 DE 3633840**

(43) Veröffentlichungstag der Anmeldung: **08.06.88 Patentblatt 88/23**

(45) Bekanntmachung des Hinweises auf die Patenterteilung: **27.02.91 Patentblatt 91/09**

(84) Benannte Vertragsstaaten: **AT BE CH DE ES FR GB GR IT LI NL SE**

(56) Entgegenhaltungen:
**EP-A- 0 151 866**
**EP-A- 0 204 242**
**EP-A- 0 234 119**
**DE-A- 1 670 382**

(73) Patentinhaber: **HOECHST AKTIENGESELLSCHAFT**
**Postfach 80 03 20**
**D-6230 Frankfurt am Main 80(DE)**

(72) Erfinder: **Sohn, Erich, Dr.**
**Lerchenbergstrasse 46/1**
**D-7300 Esslingen(DE)**
Erfinder: **Handte, Reinhard, Dr.**
**Theilweg 23**
**D-8901 Gablingen(DE)**
Erfinder: **Mildenberger, Hilmar, Dr.**
**Fasanenstrasse 24**
**D-6233 Kelkheim (Taunus)(DE)**
Erfinder: **Bürstell, Helmut, Dr.**
**Am Hohlacker 65**
**D-6000 Frankfurt am Main 50(DE)**
Erfinder: **Bauer, Klaus, Dr.**
**Doorner Strasse 53D**
**D-6450 Hanau(DE)**
Erfinder: **Bieringer, Hermann, Dr.**
**Eichenweg 26**
**D-6239 Eppstein/Taunus(DE)**

EP 0 269 806 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Phenylaminopyrazole mit herbizider Wirkung sind z.B. aus EP-A 138 149 bekannt. Aus DE-A 16 70 382 sind substituierte N-Phenylpyrazol-5-carbonsäuren bekannt, die als Zwischenprodukte zur Herstellung von Penicillansäurederivaten eingesetzt werden.

Es wurden neue Phenylpyrazolcarbonsäurederivate gefunden die überraschenderweise hervorragende pflanzenwachstumsregulierende Eigenschaften besitzen und darüberhinaus phytotoxische Nebenwirkungen von Herbiziden gegenüber Kulturpflanzen vermindern.

Gegenstand der vorliegenden Erfindung sind daher die Verbindungen der Formel I

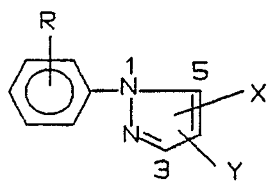

(I),

worin

R unabhängig voneinander Halogen, Hydroxy, Cyano, Nitro, $(C_1-C_4)$Alkyl, $(C_1-C_4)$Halogenalkyl, $(C_1-C_4)$-Alkoxy-$(C_1-C_4)$alkyl, $(C_1-C_6)$Alkoxy, $(C_1-C_6)$Alkoxy-$(C_1-C_4)$alkoxy, $(C_1-C_6)$Halogenalkoxy, $(C_1-C_4)$Alkylthio, $(C_1-C_4)$Halogenalkylthio, Carboxy, $(C_1-C_4)$Alkoxycarbonyl, $(C_1-C_4)$Alkylsulfinyl, $(C_1-C_4)$Halogenalkylsulfinyl, $(C_1-C_4)$Alkylsulfonyl, $(C_1-C_4)$Halogenalkylsulfonyl, $(C_1-C_4)$-Alkylsulfonyloxy, Halogen$(C_1-C_4)$alkylsulfonyloxy, Phenyl, Halogenphenyl, Phenoxy oder Halogenphenoxy,

X = in Position 3 oder 5 des Pyrazolringes orientiert ist und einen Rest der Formeln

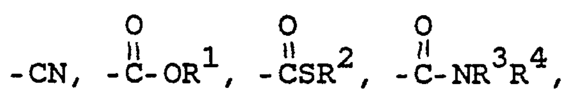

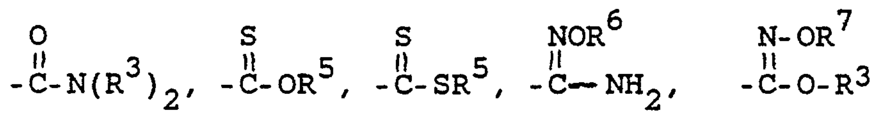

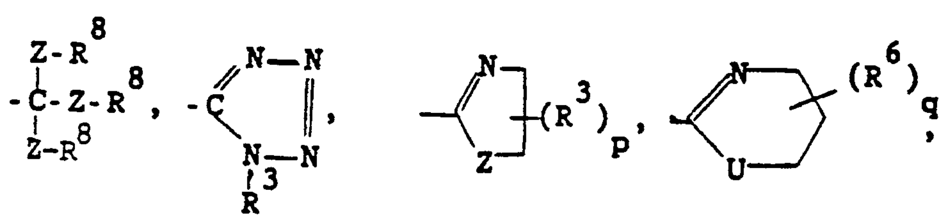

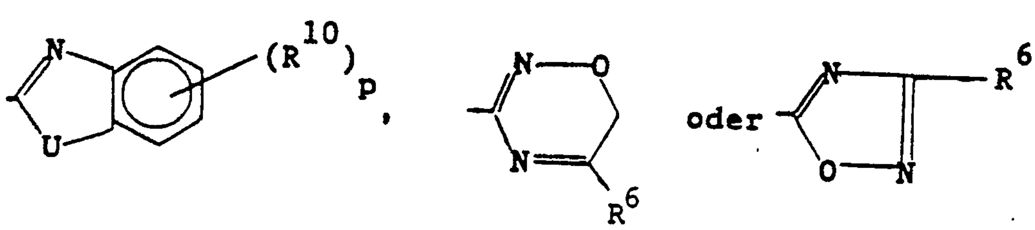

Y = Halogen

Z = O oder S

U = O, S oder N-$R^6$,

$R^1$ Wasserstoff, $(C_1-C_{12})$Alkyl, $(C_1-C_{12})$Alkyl, das ein- oder mehrfach durch Halogen und/oder ein- bis zweifach durch Hydroxy, $(C_1-C_6)$Alkoxy, $(C_1-C_4)$Akoxy$(C_1-C_4)$alkoxy, $(C_1-C_4)$-Alkylthio, $(C_1-C_4)$-Alkylsulfinyl, $(C_1-C_4)$Alkylsulfonyl, Mono- ode Di-$(C_1-C_4$-alkyl)amino, Cyano, Aminocarbonyl, $(C_1-C_4)$-Alkylcarbonyl, $(C_1-C_4$-Alkoxy)carbonyl, Cyclo$(C_3-C_7)$-alkyl, Tri$(C_1-C_4)$alkyl-silyl, Benzyloxy, Benzyloxyethoxy, Phenyl, Phenyl, das durch Halogen oder $(C_1-C_4)$Alkyl substituiert ist, durch Phenoxy, Phenylthio,

die durch Halogen oder ($C_1$-$C_4$)-Alkyl substituiert sein können, durch Oxiranyl, Tetrahyrofuryl, Triazolyl, Pyridinyl, Imidazolyl, durch Carboxy, Carboxylat mit einem für die Landwirtschaft einsetzbaren Kation oder durch den Rest $-O-N=C(CH_3)_2$ substituiert ist, ($C_3$-$C_6$)Alkenyl, ($C_3$-$C_6$)-Halogenalkenyl, unsubstituiertes oder durch Halogen oder ($C_1$-$C_4$)Alkyl substituiertes Cyclo($C_3$-$C_7$)alkyl, unsubstituiertes oder durch Halogen oder ($C_1$-$C_4$)Alkyl substituiertes Cyclo($C_5$-$C_7$)alkenyl, ($C_3$-$C_6$)Alkinyl, 1,2-Epoxy-prop-3-yl, Phenyl oder Phenyl, das ein oder zweifach durch Halogen, Nitro, Cyano, ($C_1$-$C_4$)Alkyl, ($C_1$-$C_4$-Alkoxy)-carbonyl oder ($C_1$-$C_4$)Alkoxy substituiert ist, ($C_1$-$C_4$-Alkyl)carbonyl, Phenylcarbonyl, wobei der Phenylring durch Halogen, Nitro, Cyano oder ($C_1$-$C_4$)Alkyl substituiert sein kann, einen Rest der Formeln

$-N=C(R^{10})_2$ , $-NR^3R^{11}$, 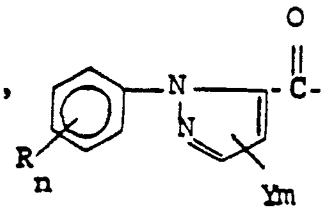 , 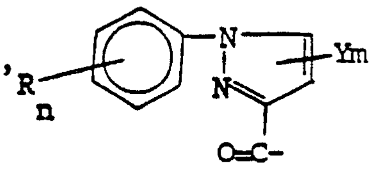

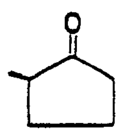 , 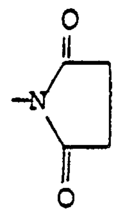 , 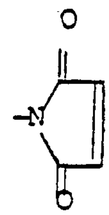 , 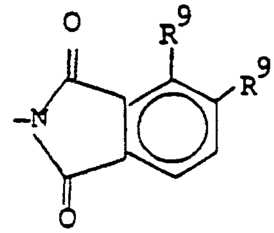

oder ein für die Landwirtschaft einsetzbares Kation,

$R^2$ ($C_1$-$C_{12}$)Alkyl oder ($C_1$-$C_{12}$)Alkyl, das bis zu zweifach durch ($C_1$-$C_4$)Alkoxyethoxy , Cyclo($C_3$-$C_6$)alkyl, Benzyloxy, Phenyl, Phenoxy, ($C_1$-$C_4$)Alkylthio, ($C_1$-$C_4$-Alkoxy)-carbonyl, Carboxy oder Carboxylat mit einem für die Landwirtschaft einsetzbares Kation, substituiert ist,

$R^3$ jeweils unabhängig voneinander ($C_1$-$C_6$)-Alkyl, Phenyl oder ($C_3$-$C_6$)-Alkenyl,

$R^4$ Wasserstoff, ($C_1$-$C_{12}$)Alkyl oder ($C_1$-$C_{12}$)Alkyl, das bis zu zweifach durch ($C_1$-$C_6$)Alkoxy, ($C_1$-$C_4$)-Alkoxy-ethoxy, Hydroxy, Hydroxyamino, ($C_1$-$C_4$)-Alkoxyimino, Halogen, Cyclo($C_3$-$C_6$)alkyl, Benzyloxy, Cyano, Aminocarbonyl, Carboxy, ($C_1$-$C_4$-Alkoxy)-carbonyl, Formyl, Phenyl oder Phenoxy substituiert ist, Phenyl oder Phenyl, das bis zu zweifach durch Halogen, Nitro, Cyano, ($C_1$-$C_4$)Alkyl oder ($C_1$-$C_4$)Alkoxy substituiert ist; ($C_3$-$C_6$)-Alkenyl, ($C_3$-$C_6$)Cycloalkyl, einen Rest der Formeln $-NR^3R^{12}$, $-O-R^6$, $-NH-CONH_2$, $-NH-CS-NH_2$ oder $-SO_2R^{13}$ oder $R^3$ und $R^4$ gemeinsam mit dem Stickstoffatom an das sie gebunden sind, einen gesättigten oder ungesättigten gegebenenfalls benzokonensierten drei- bis siebengliedrigen Ring, der bis zu drei Heteroatome aus der Gruppe O, N oder S enthält und der unsubstituiert oder durch ($C_1$-$C_4$)Alkyl oder Halogen substituert ist und eine Carbonylgruppe enthalten kann,

$R^5$ H, ($C_1$-$C_6$)Alkyl oder Phenyl, oder im Falle X = $-CS-OR^5$ ein für die Landwirtschaft einsetzbares Kation,

$R^6$ jeweils unabhängig voneinander H, ($C_1$-$C_4$)Alkyl oder Benzyl,

$R^7$ jeweils unabhängig voneinander H, ($C_1$-$C_{12}$)Alkyl, das unsubstituiert oder durch Phenyl, das unsubstituiert oder durch Halogen, Nitro, Cyano, ($C_1$-$C_4$)Alkyl oder ($C_1$-$C_4$)Alkoxy substituiert ist, durch Hydroxy, Cyano, ($C_1$-$C_4$-Alkoxy)-carbonyl, ($C_1$-$C_4$)-Alkylthio, ($C_1$-$C_4$)Alkoxy, Cyclo($C_5$-$C_7$)alkyl oder Benzyloxy substituiert ist,
($C_3$-$C_6$)Alkenyl, Halogen($C_3$-$C_6$)Alkenyl,
($C_3$-$C_6$)Alkinyl, Cyclo($C_5$-$C_8$)alkyl,
Cyclo($C_5$-$C_6$)alkenyl, ($C_1$-$C_6$-Alkyl)carbonyl,
Halogen($C_1$-$C_6$-alkyl)carbonyl,
[($C_1$-$C_6$-Alkyl)amino]carbonyl, Benzoyl, Halogenbenzoyl
oder Methylbenzoyl

$R^8$ jeweils unabhängig voneinander ($C_1$-$C_6$)Alkyl, das unsubstituiertes oder durch Phenyl, Cyclo($C_5$-$C_7$)-alkyl, ($C_1$-$C_4$)Alkoxy, ($C_1$-$C_4$)Alkylthio oder Halogen substituiert ist, oder zwei Reste $R^8$ gemeinsam mit Z und dem Kohlenstoffatom, an das sie gebunden sind, einen unsubstituierten oder durch ($C_1$-$C_4$)Alkyl, Hydroxy($C_1$-$C_4$)alkyl, Halogen($C_1$-$C_4$)alkyl oder Phenyl substituierten 5- oder 6-gliedrigen gesättigten heterocyclischen Ring;

$R^9$ jeweils unabhängig voneinander H, Halogen, ($C_1$-$C_4$)Alkyl, Nitro oder Cyano,

$R^{10}$ unabhängig voneinander H, $(C_1-C_6)$Alkyl, das unsubstituiert oder durch $(C_1-C_4)$Alkoxy, Triazolyl oder Imidazolyl substituiert ist, Cyclo$(C_3-C_6)$alkyl, $(C_3-C_6)$Alkenyl, Phenyl oder Benzyl, oder im Falle $R^1$ =-$N=C(R^{10})_2$ beide Reste $R^{10}$ gemeinsam mit dem Kohlenstoffatom, an das sie gebunden sind, ein unsubstituiertes oder durch Methyl oder Halogen substituiertes Cyclo-$(C_5-C_7)$alkyl,

$R^{11}$ $(C_1-C_4)$Alkyl, Phenyl, $(C_1-C_6$-Alkyl)carbonyl, Benzyl, Benzoyl, Halogenbenzyl, Halogenbenzoyl oder Methylbenzoyl,

$R^{12}$ H, $(C_1-C_4)$Alkyl, Formyl, $(C_1-C_6$-Alkyl)carbonyl, Benzoyl, Halogenbenzoyl, Methylbenzoyl oder Trihalogenacetyl,

$R^{13}$ $(C_1-C_4)$Alkyl, Phenyl oder Methylphenyl,

m 0 oder 1,

n eine ganze Zahl von 0 bis 5, insbesondere 1 bis 3,

p eine ganze Zahl von 0 bis 4, insbesondere 0 bis 2 und

q eine ganze Zahl von 0 bis 6, insbesondere 0 bis 3, bedeuten, sowie deren für landwirtschaftliche Zwecke verträglichen Salze und Quaternisierungsprodukte.

Die Salzbildung bzw. Quaternisierung erfolgt hierbei am basischen Stickstoffatom des Pyrazolrings. Die Salzbildung oder Quaternisierung ist nicht möglich, wenn $R^1$, $R^5$ ein Kation bedeutet oder R, $R^1$, $R^2$ eine Carboxylatgruppe enthält.

Bevorzugt unter den Verbindungen der Formel I sind insbesondere solche, bei denen R = Halogen, $(C_1-C_4)$Alkyl, Halogen$(C_1-C_4)$alkyl oder $(C_1-C_4)$Alkoxy; X = CN, -$COOR^1$, CO-$SR^2$ oder -$CONR^3R^4$; Y = Halogen; $R^1$, $R^2$ = H, $(C_1-C_4)$alkyl oder ein Kation; $R^3$, $R^4$ = H, $(C_1-C_4)$Alkyl, m = 0 oder 1 und n = 1 bis 3 bedeuten. Von besonderem Interesse hierbei sind Verbindungen mit $R_n$ = 2,6-Dialkyl, Mono- oder Diahalogen oder mono-Trifluormethyl.

Der Rest Y ist insbesondere in Position 4 des Pyrazolringes orientiert.

Unter Halogen ist F, Cl, Br oder J, insbesondere F, Cl oder Br zu verstehen.

$(C_1-C_4)$Halogenalkyl enthält 1 bis 5, insbesondere 1 bis 3 Chlor oder Fluoratome; bevorzugt ist der Rest $CF_3$.

Halogeniertes $(C_1-C_{12})$Alkyl enthält insbesondere 1 bis 13 Chlor- oder Fluoratome, hierzu zählen beispielsweise die Reste 2,2,2-Trichlorethyl, 4-Chlorbutyl, 2,2,2-Trifluorethyl, 1,1,1,3,3,3-Hexafluorprop-2-yl; 2,2,3,4,4,4-Hexafluorbutyl und 3,3,4,4,5,5,6,6,7,7,8,8,8-Tridekafluoroct-1-yl. $(C_1-C_6)$Halogenalkylthio,Halogen-$(C_1-C_4)$alkylsulfinyl, Halogen$(C_1-C_4)$alkylsulfonyl und Halogen$(C_1-C_4)$alkylsulfonyloxy enthalten jeweils insbesondere 1 bis 9 Chlor- oder Fluoratome;

Halogeniertes $(C_3-C_6)$Alkenyl enthält insbesondere 1 bis 3 Chlor oder Fluoratome.

Halogenphenyl, Halogenbenzyl oder Halogenbenzoyl enthalten insbesondere 1 bis 3 Fluor, Chlor oder Bromatome.

Unter Trihalogenacetyl ist insbesondere Trichlor- und Trifluoracetyl zu verstehen.

Für den Fall, daß der Rest -$NR^3R^4$ (für X = CO-$NR^3R^4$) einen heterocyclischen Ring bildet, ist hierunter beispielsweise Piperidin, Morpholin, 2,6-Dimethylmorpholin, Piperazin, Triazol, Imidazol, Pyrazol, Thiazol und Benzimidazol zu verstehen.

Für den Fall, daß in den aufgeführten Substituenten - zusätzlich zum Pyrazolring - weitere basische Stickstoffatome auftreten, ist auch eine mehrfache Salzbildung oder Quaternisierung möglich.

Für die Herstellung der Salze geeignet sind alle anorganischen oder organischen Säuren, die aufgrund ihres pKs-Wertes zur Salzbildung befähigt sind, z.B. Halogenwasserstoffsäuren, Salpetersäure, Schwefelsäure, Phosphorsäure, Phosphonsäuren, Sulfonsäuren, Halogenessigsäuren oder Oxalsäure.

Als Quaternisierungsprodukte sind die Umsetzungsprodukte mit Alkyl-, Alkylthioalkyl-, Alkoxyalkyl-, insbesondere $(C_1-C_6)$Alkyl- und gegebenenfalls im Phenylrest substituierten, insbesondere halogenierten Phenacylhalogeniden zu verstehen. Die Herstellung der Quaternisierungsprodukte der Verbindungen der Formel I erfolgt nach allgemein üblichen Methoden.

Als Kationen für $R^1$, $R^2$ oder $R^5$, die für die Landwirtschaft einsetzbar sind, kommen Metallkationen z.B. Alkali- oder Erdalkalikationen wie Na, K, Mg oder organische kationen wie organisches substituiertes Ammonium, organisch substituiertes Phosphonium, Sulfonium oder Sulfoxonium oder andere Stickstoffkationen in Betracht.

Organisch substituiertes Ammonium bedeutet primäres, sekundäres, tertiäres, quartäres, aliphatisches, aromatisches oder heteroaromatisches Ammonium, das 1 bis drei N- Atome enthalten kann. Die Stickstoffatome des Amins können hierbei auch Teil eines cyclischen Systems sein. Als Beispiele für solche Ammoniumsalze seien genannt: Mono-, Di-, Tri-, Tetra[$(C_1-C_6)$Alkyl]ammonium wie Isopropylammonium, Butylammonium, Stearylammonium, Triethylammonium, Mono-, Di-, Tri-[$(C_1-C_4)$alkoxy$(C_1-C_4)$alkyl]-ammonium oder Mono-, Di-, Tri-[$(C_1-C_6)$-alkanol]-ammonium wie Methoxyethylammonium, Methoxypropylammonium, Triethanolammonium, Tripropanolammonium, oder Ammoniumverbindungen mit gemischten

Reste wie tert.-Butyldiethanolammonium, Triethylbenzylammonium, Hydroxyethyltrimethylammonium, Chlorethyltrimethylammonium, oder Allylammonium, Diallyammonium, Cyclohexylammonium, Menthanylammonium, Aminoethylammonium, Ethylendiammonium, Benzhydrylammonium, Pyrrolidinium, Morphilinium, 3-Pyridylammonium, Piperidinium oder Piperazinium, oder ein von einer Aminosäure oder deren Ester abgeleitetes Ammonium wie $[NH_3\text{-}CH_2\text{-}COOCH_3]^+$.

Organisch substituiertes Phosphonium, organisches Sulfonium oder organisches Sulfoxonium enthalten aliphatische oder arylaliphatische Reste, wie sie für Ammonium angegeben wurden.

Andere Stickstoff-Kationen sind beispielsweise Hydrazonium, Hydroxylammonium, Guanidinium, Aminoguanidinium oder deren Substitutionsprodukte.

Gegenstand der Erfindung ist ferner ein Verfahren zur Herstellung der Verbindungen der Formel I, dadurch gekennzeichnet, daß man eine Verbindung der Formel II

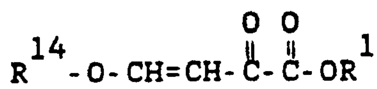

(II),

worin $R^{14}$ $(C_1\text{-}C_6)$-Alkyl bedeutet, mit einer Verbindung der Formel III

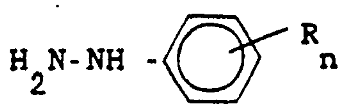

(III)

umsetzt und anschließend gegebenenfalls derivatisiert.

Das Verfahren wird bei 0° bis 120° C in einem organischen Lösemittel gegebenenfalls in Gegenwart einer organischen Säure, wie p-Toluolsulfonsäure, Methansulfonsäure, durchgeführt. Als Lösemittel können polare Verbindungen wie Alkohole, z.B. Ethanol, Methanol, organische Säuren wie Eisessig, chlorierte Kohlenwasserstoffe wie Dichlorethan oder aromatische Lösemittel wie Toluol, Xylol eingesetzt werden.

Während der Reaktion entstehen als Zwischenstufen die Verbindungen der Formel IVa und IVb.

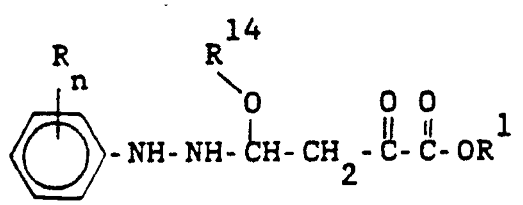

(IVa)

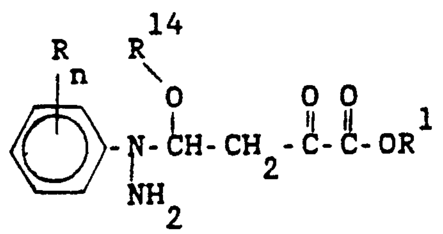

(IVb)

Diese Zwischenprodukte können isoliert werden und anschließend unter den oben beschriebenen Bedingungen cyclisiert werden. Bei der direkten Weiterreaktion werden in der Regel Gemische der Verbindungen der Formel I d.h. die Verbindungen der Formel Ia und Ib nebeneinander erhalten.

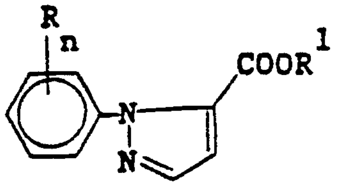

(Ia)

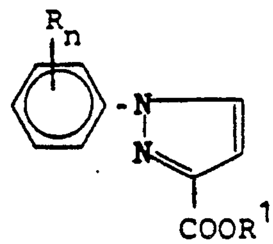

(Ib)

Die Verbindungen der Formeln (Ia) bzw. (Ib) können nach üblichen Verfahren an der Gruppe $-COOR^1$ oder durch Halogenierung des Pyrazolrestes derivatisiert werden.

So lassen sich die Pyrazole der Formeln Ia oder Ib unter den üblichen Bedingungen der Aromatenhalogenierung in der 4-Position des Pyrazolrestes halogenieren, s. Houben-Weyl, Methoden der organischen Chemie Band 5/3 S. 503 ff, Band 5/4, S. 13 ff (1962). Zur Derivatisierung wird weiterhin der Rest- $-COOR^1$ in bekannter Weise in andere für X genannte Reste ungewandelt, z.B. durch Verseifung, Veresterung, Umesterung, Amidierung, Salzbildung etc. wie dies z.B. in den deutschen Offenlegungsschriften DE-OS 34 44 918 und DE-OS 34 42 690 beschrieben ist, oder es erfolgt auf übliche Weise Salzbildung oder Quaternisierung am basischen Stickstoffatoms des Pyrazolrings.

Die Ausgangsverbindungen der Formel II lassen sich durch Umsetzung der Verbindungen der Formel V mit Verbindungen der Formel VI in Gegenwart einer organischen Hilfsbase,

$$R^{14}-OCH=CH_2 \qquad (V)$$

$$R^{15}-\overset{\overset{O}{\|}}{C}-\overset{\overset{O}{\|}}{C}-OR^{1} \qquad (VI)$$

erhalten (Literatur; Chem. Ber. 115, S. 2766-2782 (1982)). $R^{15}$ bedeutet eine Abgangsgruppe wie Cl, Br, $OSO_2CF_3$

Als Hilfsbase können organische Amine wie Triethylamin oder Pyridin eingesetzt werden. Das Verfahren wird zwischen $-20^\circ$ und $+30^\circ C$ durchgeführt. Die erhaltenen Verbindungen der Formel II können direkt ohne Aufarbeitung weiter umgesetzt werden. Die Ausgangsverbindungen der Formel III lassen sich nach üblichem Verfahren, s. Houben Weyl, Methoden der organischen Chemie Bd 10/2 S. 169 (1967) herstellen.

Gegenstand der Erfindung ist ferner die Verwendung der Verbindungen der Formel I als Pflanzenwuchsregulatoren. Mit den erfindungsgemäßen Verbindungen sind typische wachstumsregulierende Effekte erzielbar. Die Verbindungen greifen regulierend in den pflanzeneigenen Stoffwechsel ein und können damit zur gezielten Beeinflussung von Pflanzeninhaltsstoffen sowie zur Ernteerleichterung wie zum Auslösen von Desikkation und Wuchsstauchung eingesetzt werden. Des weiteren eignen sie sich zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann. Besonders hervorzuheben ist die wachstumsregulatorische Wirksamkeit der Verbindungen als Wuchshemmer in Getreide, Mais, Soja, Tabak, Baumwolle, Ackerbohne, Raps, Reis, Sonnenblume, Rasen sowie ihre Fähigkeit, den Gehalt an erwünschten Inhaltsstoffen wie Kohlehydraten (z.B. Zuckerrohr oder Hirsekulturen) und Protein bei Nutzpflanzen zu erhöhen. Schließlich zeigen die Verbindungen eine sehr gute Verbesserung der Fruchtabszission, insbesondere bei Zitrusfrüchten.

Eine weitere Lösung der gestellten Aufgabe sind auch das Pflanzenwachstum regulierende Mittel, die sich durch einen wirksamen Gehalt mindestens einer der erfindungsgemäßen Verbindung auszeichnen. Die Aufwandmenge der Verbindungen der Formel I beträgt im allgemeinen 0,02 bis 2,5 kg Wirksubstanz pro ha, vorzugsweise 0,05 bis 1,5 kg/ha. Die Verbindungen lassen sich bei ihrem praktischen Einsatz gegebenenfalls auch vorteilhaft mit bekannten Wachstumsregulatoren oder natürlichen oder pflanzlichen Hormonen kombinieren.

Gegenstand der Erfindung ist ferner die Verwendung der Verbindungen der Formel I als Safener. So wurde gefunden, daß sie phytotoxische Nebenwirkungen von Pflanzenschutzmitteln, insbesondere von

Herbiziden, beim Einsatz in Nutzpflanzenkulturen vermindern oder ganz unterbinden.

Die Verbindungen der Formel I können zusammen mit anderen Herbiziden ausgebracht werden und sind dann in der Lage, schädliche Nebenwirkungen dieser Herbizide zu antagonisieren oder völlig aufzuheben, ohne die herbizide Wirksamkeit dieser Herbizide gegen Schadpflanzen zu beeinträchtigen. Hierdurch kann das Einsatzgebiet herkömmlicher Pflanzenschutzmittel ganz erheblich vergrößert werden. Solche Verbindungen, die die Eigenschaft besitzen, Kulturpflanzen gegen phytotoxische Schäden durch Herbizide zu schützen, werden Antidots oder "Safener" genannt.

Herbizide, deren phytotoxische Nebenwirkungen mittels der Verbindungen der Formel I herabgesetzt werden können, sind z.B. Carbamate, Thiolcarbamate, Halogenacetanilide, substituierte Phenoxy-, Naphthoxy- und Phenoxyphenoxycarbonsäurederivate sowie Heteroaryloxyphenoxycarbonsäurederivate wie Chinolyloxy-, Chinoxalyloxy-, Pyridyloxy-, Benzoxazolyloxy-, Benzthiazolyloxy-phenoxy-carbonsäureesteer und ferner Dimedonoximabkömmlinge. Bevorzugt hiervon sind Phenoxyphenoxy- und Heteroaryloxyphenoxycarbonsäureester. Als Ester kommen hierbei insbesondere niedere Alkyl-, Alkenyl und Alkinylester in Frage.

Beispielsweise seien, ohne daß dadurch eine Beschränkung erfolgen soll, folgende Herbizide genannt:

A) Herbizide vom Typ der Phenoxyphenoxy- und Heteroaryloxy phenoxycarbonsäure-($C_1$-$C_4$)alkyl-, ($C_2$-$C_4$)alkenyl- und ($C_3$-$C_4$)alkinylester wie
2-(4-(2,4-Dichlorphenoxy)-phenoxy)-propionsäuremethylester,
2-(4-(4-Brom-2-chlorphenoxy)-phenoxy)-propionsäuremethylester,
2-(4-(4-Trifluormethylphenoxy)-phenoxy)-propionsäuremethylester,
2-(4-(2-Chlor-4-trifluormethylphenoxy)-phenoxy)-propionsäuremethylester,
2-(4-(2,4-Dichlorbenzyl)-phenoxy)-propionsäuremethylester,
4-(4-(4-Trifluormethylphenoxy)-phenoxy)-pent-2-en-säureethylester.
2-(4-(3,5-Dichlorpyridyl-2-oxy)-phenoxy)-propionsäureethylester,
2-(4-(3,5-Dichlorpyridyl-2-oxy)-phenoxy)-propionsäurepropargylester,
2-(4-(3,5-Dichlorpyridyl-2-oxy)-phenoxy)-propionsäuretrimethylsilylmethylester,
2-(4-(6-Chlorbenzoxazol-2-yl-oxy)-phenoxy)-propionsäureethylester,
2-(4-(6-Chlorbenzthiazol-2-yl-oxy)-phenoxy)-propionsäureethylester,
2-(4-(3-Chlor-5-trifluormethyl-2-pyridyloxy)-phenoxy)-propionsäuremethylester,
2-(4-(3-Chlor-5-trifluormethyl-2-pyridyloxy)-phenoxy)-propionsäureethylester
2-(4-(5-Trifluormethyl-2-pyridyloxy)-phenoxy)-propionsäurebutylester,
2-(4-(6-Chlor-2-chinoxalyloxy)-phenoxy)-propionsäureethylester,
2-(4-(6-Fluor-2-chinoxalyloxy)-phenoxy)-propionsäureethylester,
2-(4-(6-Chlor-2-chinolyloxy)-phenoxy)-propionsäureethylester,

B) Chloracetanilid-Herbizide wie
N-Methoxymethyl-2,6-diethyl-chloracetanilid,
N-(3'-Methoxyprop-2'-yl)-methy-6-ethyl-chloracetanilid,
N-(3-Methyl-1,2,4-oxdiazol-5-yl-methyl)-chloressigsäure-2,6-dimethylanilid,

C) Thiocarbamate wie
S-Ethyl-N,N-dipropylthiocarbamat oder
S-Ethyl-N,N-diisobutylthiocarbamat

D) Dimedon-Derivate wie
2-(N-Ethoxybutyrimidoyl)-5-(2-ethylthiopropyl)-3-hydroxy 2-cyclo-hexen-1-on,
2-(N-Ethoxybutyrimidoyl)-5-(2-phenylthiopropyl)-3-hydroxy-2-cyclohexen-1-on oder
2-(1-Allyloxyiminobutyl)-4-methoxycarbonyl-5,5-dimethyl-3-oxocyclohexenol.
2-(N-Ethoxypropionamidoyl)-5-mesityl-3-hydroxy-2-cyclohexen-1-on,
2-(N-Ethoxybutyrimidoyl)-3-hydroxy-5-(thian-3-yl)-2-cyclohexen-1-on.

Das Mengenverhältnis Safener : Herbizid kann innerhalb weiter Grenzen, im Bereich zwischen 1 : 10 und 10 : 1, insbesondere zwischen 2 : 1 und 1 : 10, schwanken. Die jeweils optimalen Mengen an Herbizid und Safener sind abhängig vom Typ des verwendeten Herbizids oder vom verwendeten Safener sowie von der Art des zu behandelnden Pflanzenbestandes und lassen sich von Fall zu Fall durch entsprechende Versuche ermitteln.

Haupteinsatzgebiete für die Anwendung der Safener sind vor allem Getreidekulturen (Weizen, Roggen, Gerste, Hafer), Reis, Mais, Sorghum, aber auch Baumwolle, Zuckerrüben, Zuckerrohr und Sojabohne.
Die Safener der Formel I können je nach ihren Eigenschaften zur Vorbehandlung des Saatgutes der Kulturpflanze (Beizung der Samen) verwendet werden oder vor der Saat in die Saatfurchen eingebracht werden oder zusammen mit dem Herbizid vor oder nach dem Auflaufen der Pflanzen angewendet werden. Vorauflaufbehandlung schließt sowohl die Behandlung der Anbaufläche vor der Aussaat als auch die

Behandlung der angesäten, aber noch nicht bewachsenen Anbauflächen ein. Bevorzugt ist die gemeinsame Anwendung mit dem Herbizid. Hierzu können Tankmischungen oder Fertigformulierungen eingesetzt werden.

Gegenstand der vorliegenden Erfindung ist daher auch ein Verfahren zum Schutz von Kulturpflanzen vor phytotoxischen Nebenwirkungen von Herbiziden, das dadurch gekennzeichnet ist, daß eine wirksame Menge einer Verbindung der Formel I vor, nach oder gleichzeitig mit dem Herbizid appliziert wird.

Die erfindungsgemäßen Verbindungen der Formel I können, gegebenenfalls im Gemisch mit weiteren Wirkkomponenten oder auch zusammen mit einem Herbizid, als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Unter Spritzpulvern werden in Wasser gleichmäßig dispergierbare Präparate verstanden, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Ihre Herstellung erfolgt in üblicher Weise z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil ganz oder auch teilweise entfallen. Als Emulgatoren können beispielsweise verwendet werden: Alkylarylsulfonsaure Calciumsalze wie Ca-dodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel werden durch Vermahlen des Wirkstoffes mit fein verteilten, festen Stoffen, z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration etwa 10 bis 90 Gew.-%; der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubfähige Formulierungen enthalten meistens 5 bis 20 Gew.-% an Wirkstoff, versprühbare Lösungen etwa 1 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll-oder Trägerstoffe.

Zur Anwendung werden in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Die benötigten Aufwandmengen der Verbindungen der Formel I bei ihrem Einsatz als Safener können je nach Indikation und verwendetem Herbizid innerhalb weitere Grenzen schwanken und variieren im allgemeinen zwichen 0,01 und 10 kg Wirkstoff je Hektar.

Folgende Beispiele dienen zur Erläuterung der Erfindung.

A. Formulierungsbeispiele

a) Ein Stäbemittel wird erhalten, indem man 10 Gew.-Teile einer Verbindung der Formel I und 90 Gewichtsteile Talkum oder Inertstoff mischt und in einer Schlagmühle zerkleinert.

b) Ein in Wasser leicht dispergierbares, benetzbares Pulver wird erhalten, indem man 25 Gewichtsteile einer Verbindung der Formel I, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gewichtsteile einer Verbindung der Formel I mit 6 Gewichtsteilen Alkylphenolpolyglykolether ((R)Triton X 207), 3 Gewichtsteilen Isotridecanolpolyglykolether (8 AeO) und 71 Gewichtsteilen paraffinischem Mineralöl (Siedebereich z.B. ca. 255 bis über 377 °C) mischt und in einer Reibkugelmühle auf eine Feinheit von unter 5 Mikron vermahlt.

d) Ein emulgierbares Konzentrat wird erhalten aus 15 Gewichtsteilen einer Verbindung der Formel I, 75 Gewichtsteilen Cyclohexanon als Lösungsmittel und 10 Gewichtsteilen oxethyliertes Nonylphenol als Emulgator.

e) Ein Wasser leicht emulgierbares Konzentrat aus einem Phenoxycarbonsäureester und einem Antidot (10 : 1) wird erhalten aus

12,00 Gew.-% 2-[4-(6-Chlorbenzoxazol-2-yl-oxy)-phenoxy]-propionsäureethylester
1,20 Gew.-% Verbindung der Formel I
69,00 Gew.-% Xylol
7,80 Gew.-% dodecylbenzolsulfonsaurem Calcium
6,00 Gew.-% ethoxyliertem Nonylphenol (10 EO)
4,00 Gew.-% ethoxyliertem Rizinusöl (40 EO)

Die Zubereitung erfolgt wie unter Beispeil a) angegeben.

f) Ein in Wasser leicht emulgierbares Konzentrat aus einem Phenoxycarbonsäureester und einem Antidot (1 : 10) wird erhalten aus

4,0 Gew.-% 2-[4-(6-Chlorbenzoxazol-2-yl-oxy)-phenoxy]-propionsäureethylester
40,0 Gew.-% Verbindung der Formel I
30,0 Gew.-% Xylol
20,0 Gew.-% Cyclohexanon
4,0 Gew.-% dodecylbenzolsulfonsaurem Calcium
2,0 Gew.-% ethoxyliertem Rizinusöl (40 EO)

B. Chemische Beispiele

Beispiele 1 und 2

1-Phenyl-pyrazol-5(und 3)-carbonsäureethylester

Zu 14 g Oxalsäurehalbethylesterchlorid wurde zwischen 0° und 30 °C 15 g Ethylvinylether zugetropft und 20 h bei 20 - 30 °C nachgerührt. Das Reaktionsgemisch wurde im Wasserstrahlvakuum eingeengt und in 100 ml Eisessig aufgenommen. Zu dieser Lösung tropfte man zwischen 10 und 80 °C 10,8 g Phenylhydrazin in 150 ml Eisessig zu und erhitzte das Gemisch 2 h zum Rückfluß. Man gab das erhaltene Produkt in 1 l Wasser und extrahierte es zweimal mit 300 ml Essigester. Der organische Extrakt wurde einmal mit 100 ml Wasser, zweimal mit 100 ml gesättigter $NaHCO_3$-Lösung und wieder mit 100 ml Wasser gewaschen und über $Mg_2SO_4$ getrocknet. Nach destillativer Trennung erhielt man 1-Phenyl-pyrazol-5-carbonsäureethylester Kp 100-102/0,5 Torr (Beispiel 1) 1-Phenyl-pyrazol-3-carbonsäureethylester Kp 125-128/0,5 Torr (Beispiel 2)
Ausbeute: 10,5 g

Beispiel 3

1-Phenyl-pyrazol-5-carbonsäure

4,4 g 1-Phenyl-pyrazol-5-carbonsäureethylester von Beispiel 1 wurden mit 10 ml 16,5 % wäßrigem NaOH und 10 ml Ethanol 6 h bei Raumtemperatur gerührt; das Ethanol wurde abdestilliert, die wäßrige Phase zweimal mit 10 ml Toluol extrahiert und mit konz. HCl auf pH 3 eingestellt. Der Niederschlag wurde abgesaugt, mit wenig Wasser gewaschen und getrocknet: Man erhielt 3,1 g Produkt vom Fp. 182 - 183 °C.

Beispiel 4

1-(2,6-Dichlorphenyl)-pyrazol-5-carbonsäureethylester

Zur 137 g Oxalsäurehalbethylesterchlorid tropfte man unter Kühlen mit Eis/Kochsalz 145 g Ethylvinylether zu; Nach Erwärmen auf Raumtemperatur wurde 20 h nachgerührt. Die flüchtigen Bestandteile wurden abdestilliert und der Rückstand im Wasserstrahlvakuum fraktioniert. Man erhielt 4-Ethoxy-2-oxo-but-3-en-säureethylester vom $Kp_{13}$ 140-143 °C. 17,5 g des Produktes wurde in 200 ml Toluol gelöst. Bei 0 °C wurden 17,5 g 2,6-Dichlorphenylhydrazin unter Rühren hinzugeführt. Man erhitzte langsam zum Sieden und trennte am Wasserabscheider Ethanol und Wasser ab, bis der Siedepunkt bei 111 °C konstant blieb. Der Rückstand wurde mit Toluol verdünnt, zweimal mit 2n Salzsäure, gesättigter Hydrogencarbonatlösung und Wasser gewaschen, getrocknet, zur Trockene eingedampft und aus Ethanol umkristallisiert.
Ausbeute: 18,3 g
Fp: 51-53 °C

Beispiel 5

4-Brom-1-(2,6-Dichlorphenyl)-pyrazol-5-carbonsäureethylester

14,3 g 1-(2,6-Dichlorphenyl)-pyrazol-5-carbonsäureethylester vom Beispiel 4 wurden in 100 ml Eisessig gelöst, mit 10 g Na-Acetat versetzt und bei Raumtemperatur 4,5 g Brom zugetropft. Nach 60 h wurde das Reaktionsgemisch auf 1 l Wasser gegossen. Der Niederschlag wurde abgesaugt, mit Wasser nachgewaschen und aus Ethanol umkristallisiert. Ausbeute: 8,2 g
Fp: 62-65 °C.

Beispiele 6 und 7

1-(3-Trifluormethylphenyl)-pyrazol-5(und 3)-carbonsäurecyclohexylester

Zu 19,5 g Oxalsäurehalbcyclohexylesterchlorid wurden 15 g Ethylvinylether bei 0 °C zugetropft, das Gemisch 20 h bei Raumtemperatur gerührt und die leichtflüchtigen Anteile abdestilliert. Man gab 200 ml Toluol und 0,5 g p-Toluolsulfonsäure hinzu und erhitzte 2 h am Wasserabscheider. Bei 100 °C wurden eine Lösung von 17,6 g 3-Trifluormethylphenylhydrazin in 100 ml Toluol hinzugefügt und das Gemisch am Wasserabscheider erhitzt, bis das Destillat konstant bei 111 °C überdestillierte. Das Produkt wurde mit Toluol verdünnt, zweimal mit 100 ml 2n HCl, zweimal mit 100 ml gesättigter $NaHCO_3$ -Lösung und einmal mit 100 ml Wasser gewaschen, über $MgSO_4$ getrocknet und die Lösung zur Trockene eingedampft. Nach Säulenchromatographie erhielt man
1-(3-Trifluormethylphenyl)-pyrazol-5-carbonsäurecyclohexylester, als farbloses Oel, Ausbeute 8,2 g (Beispiel 6) und 1-(3-Trifluormethylphenyl)-pyrazol-3-carbonsäurecyclohexylester, als Oel, Ausbeute 8,7 g (Beispiel 7) Die Verbindungen wurden $^1H$-NMR-spektroskopisch charakterisiert.

Beispiele 8 und 9

1-(4-Methylphenyl)-pyrazol-5(und 3)-carbonsäuremethylester

Zu einer Lösung von 16 g 4-Ethoxy-2-oxo-but-3-en-säuremethylester in 100 ml Eisessig wurden bei 50 °C 12,5 g p-Tolylhydrazin in 150 ml Eisessig zugegeben. Man rührte 5 h bei 100 °C, gab das Gemisch auf 1 l Wasser und extrahierte zweimal mit 150 ml Essigester. Die organische Phase wurde mit gesättigter $NaHCO_3$-Lösung und anschließend mit Wasser gewaschen und getrocknet. Nach Einengen im Wasserstrahlvakuum wurde das Gemisch im Hochvakuum destillativ getrennt. Man erhielt 4,1 g
1-(4-Methylphenyl)-pyrazol-5-carbonsäure-methylester vom $Kp_{0,01}$ 116-120 °C (Beispiel 8)
und 5,3 g 1-(4-Methylphenyl)-pyrazol-3-carbonsäuremethylester vom $Kp_{0,01}$ 138-142 °C
Die Verbindungen wurden $^1H$-NMR-spektroskopisch charakterisiert.

Die in der nachfolgenden Tabelle angegebenen Verbindungen der Formel I werden nach den in den

vorangehenden Beispielen beschriebenen Verfahrensweisen hergestellt oder aus oben beschriebenen Verbindungen durch Derivatisierung erhalten.

Tabelle I

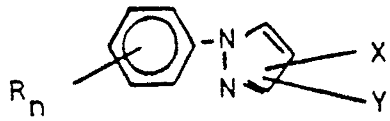

| Bsp.Nr. | $R_n$ | 4-Y | X | Fp(°C) |
|---|---|---|---|---|
| 10 | H | H | 5-COOK | |
| 11 | H | H | 5-COONa | |
| 12 | " | " | $5\text{-}COO^{\ominus}NH^{\oplus}(C_2H_4OH)_3$ | 131-132 |
| 13 | " | " | $5\text{-}COO^{\ominus}NH_3^{\oplus}\text{-}c\text{-}C_6H_{11}$ | |
| 14 | " | Br | $5\text{-}COOC_2H_5$ | 59-61 |
| 15 | H | Br | $3\text{-}COOC_2H_5$ | 75-87 |
| 16 | " | Br | $5\text{-}COO^{\ominus}$ $H_2N^{\oplus}$ 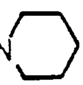 | Oel |
| 17 | H | Br | $5\text{-}COO^{\ominus}H_2N^{\oplus}\text{-}c\text{-}C_6H_{11}$ | 139-143 |
| 18 | H | Cl | $5\text{-}COOC_2H_5$ | |
| 19 | " | " | 5-COOH | |
| 20 | " | " | $5\text{-}COO\text{-}n\text{-}C_{12}H_{25}$ | |
| 21 | " | H | 3-COOH | 142-144 |
| 22 | " | " | $3\text{-}COO^{\ominus}NH(C_2H_4OH)_3$ | Oel |
| 23 | " | Br | $3\text{-}COOC_2H_5$ | |
| 24 | " | " | 3-COOH | |
| 25 | " | " | $3\text{-}COOnC_6H_{13}$ | |
| 26 | " | Cl | $3\text{-}COOCH_3$ | 66-68 |
| 27 | " | " | 3-COOH | 174-175 |
| 28 | " | " | 3-COOK | |
| 29 | " | " | $3\text{-}COOCH_2CCl_3$ | |
| 30 | $4\text{-}CH_3$ | H | 5-COOH | 192-196 |
| 31 | $4\text{-}CH_3$ | H | 3-COOH | 169-172 |
| 32 | " | Br | $5\text{-}COOC_2H_5$ | |
| 33 | " | " | $3\text{-}COOC_2H_5$ | |
| 34 | $2,4\text{-}Cl_2$ | H | $5\text{-}COOC_2H_5$ | 56-60 |
| 35 | " | " | 5-COOH | 212-213 |

| Beispiel-Nr. | $R_n$ | 4-Y | X | Fp(°C) |
|---|---|---|---|---|
| 36 | $2,4-Cl_2$ | H | 3-COOH | 177-180 |
| 37 | " | " | $5-COSC_2H_5$ | Oel |
| 38 | " | " | 5-CO 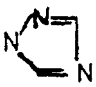 | Oel |
| 39 | " | Br | $5-COOC_2H_5$ | 45-48 |
| 40 | " | " | $3-COOC_2H_5$ | 91-102 |
| 41 | " | " | 3-COOH | 184-188 |
| 42 | " | " | 5-COOH | 175-177 |
| 43 | " | " | $5-COO^{\ominus}NH^{\oplus}(C_2H_4OH)_3$ | 72-75 |
| 44 | " | " | 5-COOK | > 260 |
| 45 | " | H | $3-COOC_2H_5$ | 72-77 |
| 46 | " | H | $5-COOCH_2CF_2CFHCF_3$ | Oel |
| 47 | " | " | $5-COO-n-C_{12}H_{25}$ | Oel |
| 48 | " | " | $5-COO-c-C_6H_{11}$ | Oel |
| 49 | " | " | $5-COO^- Li^+$ | >260 |
| 50 | " | " | $3-COO^- K^+$ | >260 |
| 51 | " | " | $5-COO^- Ca^{2+}_1/_2$ | 178-180 |
| 52 | " | " | $5-COO^{\ominus}NH_4^{\oplus}$ | 140-143 |
| 53 | " | Br | $5-CONH_2$ | 118-120 |
| 54 | " | H | $3-COO^{\ominus}NH_4^{\oplus}$ | 212-215 |
| 55 | " | Br | 5-CN | 106-110 |
| 56 | " | " | 5CO- 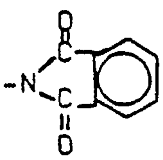 | |
| 57 | " | " | $5-CONHCH_2CH_2OH$ | 49-50 |
| 58 | " | " | $5-COOCH_2SCH_3$ | |
| 59 | " | Cl | 5- 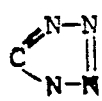 | |
| 60 | " | " | 3- 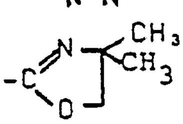 | |

| Beispiel-Nr. | $R_n$ | 4-Y | X | Fp(°C)<br>Kp (°C/Torr)<br>[°C/mbar] |
|---|---|---|---|---|
| 61 | $2,4-Cl_2$ | Cl | 5-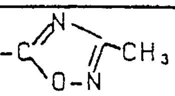 | |
| 62 | $2,6-(CH_3)_2$ | H | 5-COOH | 167-170 |
| 63 | " | " | $5-COOC_2H_5$ | (101-108/0,02)<br>[101-108/0,027] |
| 64 | " | " | $3-COO^{\ominus}\,\overset{+}{N}H(C_2H_4OH)_3$ | 83-86 |
| 65 | " | " | $3-COO^{\ominus}H_3N^{\oplus}-c-C_6H_{11}$ | 144-146 |
| 66 | " | Br | 5-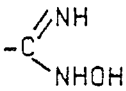 | |
| 67 | " | Br | $5-COOCH_2-CF_2CHFCF_3$ | |
| 68 | " | Cl | 5-COOH | |
| 69 | " | Cl | 5-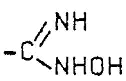 | |
| 70 | $2,6-(C_2H_5)_2$ | H | $5-COOC_2H_5$ | (119-123/0,01)<br>[119-123/0,013] |
| 71 | " | H | $3-COOC_2H_5$ | (135-152/0,01)<br>[135-152/0,013] |
| 72 | " | H | 5-COOH | 142-146 |
| 73 | " | H | 3-COOH | 162-164 |
| 74 | " | Br | 5-COOH | 117-123 |
| 75 | " | " | 3-COOH | 136-141 |
| 76 | " | " | $5-CONH_2$ | |
| 77 | " | Br | 3-CONHOH | |
| 78 | " | " | 5-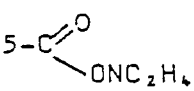 | |
| 79 | " | Cl | 5-COOH | |
| 80 | " | Cl | 3-COOH | |
| 81 | " | Cl | $5-COOn-C_{12}H_{25}$ | |
| 82 | $2-CH_3,6-C_2H_5$ | H | $5-COOC_2H_5$ | (120-125/0,02)<br>[120-125/0,027] |
| 82 | " | " | $3-COOC_2H_5$ | (140-144/0,02)<br>[140-144/0,027] |
| 83 | " | " | 5-COOH | 126-128 |
| 84 | " | " | $5-COO^{-}H_2$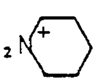 | 137-140 |

| Beispiel-Nr. | $R_n$ | 4-Y | X | Fp(°C) |
|---|---|---|---|---|
| 85 | $2\text{-}CH_3, 6\text{-}C_2H_5$ | Br | 5-COOH | |
| 86 | " | Cl | $5\text{-}COOC_2H_5$ | |
| 87 | " | " | 3-COOH | |
| 88 | $2,6\text{-}Cl_2$ | H | 5-COOH | 207-208 |
| 89 | " | Br | 5-COOH | 187-192 |
| 90 | " | H | $5\text{-}CONH_2$ | 117-118 |
| 91 | " | H | 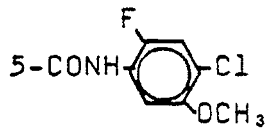 | 225 |
| 92 | " | " | $5\text{-}COSC_2H_5$ | Oel |
| 93 | " | " | $5\text{-}COO(CH_2)_2(CF_2)_5\text{-}CF_3$ | 57-61 |
| 94 | " | " | $5\text{-}COO\text{-}n\text{-}C_{12}H_{25}$ | 44-48 |
| 95 | " | " | $5\text{-}COOCH_3$ | 113-115 |
| 96 | " | " | 5-CN | 94-96 |
| 97 | " | " | $5\text{-}CONHCH_3$ | 220-223 |
| 98 | $2,6\text{-}Cl_2, 3\text{-}NO_2$ | " | $5\text{-}COOC_2H_5$ | Oel |
| 99 | " | " | 5-COOH | 178-179 |
| 100 | $2,6\text{-}Cl_2$ | " | 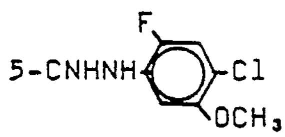 | 176-177 |
| 101 | " | " | 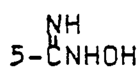 | |
| 102 | " | " | 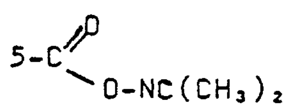 | |
| 103 | " | " | 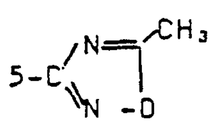 | |
| 104 | " | Br | $5\text{-}COOC_2H_5$ | |
| 105 | " | Br | $5\text{-}COOCH_2CF_2CHFCF_3$ | |
| 106 | " | " | 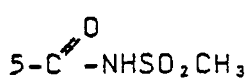 | |

| Beispiel-Nr. | $R_n$ | 4-Y | X | Fp(°C) |
|---|---|---|---|---|
| 107 | 2,6-$Cl_2$ | Br | 5-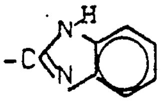 | |
| 108 | " | Cl | 5-$COOC_2H_5$ | |
| 109 | " | Cl | 5-COOH | |
| 110 | " | Cl | 5 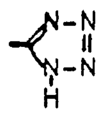 | |
| 111 | " | " | 5-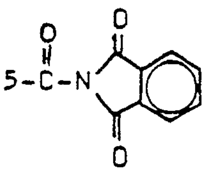 | |
| 112 | " | H | 3-COOH | |
| 113 | " | Br | 3-$COOC_2H_5$ | |
| 114 | " | Cl | 3-$COOCH_3$ | |
| 115 | 3,4-$Cl_2$ | H | 5-$COOC_2H_5$ | 95-99 |
| 116 | " | " | 3-$COOC_2H_5$ | 93-96 |
| 117 | " | " | 5-COOH | 217-219 |
| 118 | " | " | 5-$COO^{\ominus}NH^{\oplus}(C_2H_4OH)_3$ | 137-140 |
| 119 | " | Br | 5-$COONC(CH_3)_2$ | |
| 120 | " | Cl | 5-$COOCH_3$ | |
| 121 | " | " | 3-$COOC_2H_5$ | |
| 122 | " | " | 5-$COOnC_{12}H_{25}$ | |
| 123 | 3,5-$Cl_2$ | H | 5-$COOC_2H_5$ | 94-97 |
| 124 | " | " | 5-COOH | 229-232 |
| 125 | " | Br | 5-COOH | |
| 126 | " | " | 3-COOH | |
| 127 | " | Cl | 5-$COOC_2H_5$ | |
| 128 | 2,3,4-$Cl_3$ | H | 5-$COOC_2H_5$ | Oel |
| 129 | " | H | 5-COOH | 146 |
| 130 | " | " | 3-$COOC_2H_5$ | Oel |
| 131 | " | Br | 5-COOH | |
| 132 | " | " | 5-$COOCH_2CF_2CHFCF_3$ | ... |

| Beispiel-Nr. | $R_n$ | 4-Y | X | Fp(°C) |
|---|---|---|---|---|
| 133 | 2,3,4-$Cl_3$ | Br | 5-$COOCH_2CCl_3$ | |
| 134 | " | Cl | 5-COOH | |
| 135 | 2,4,6-$Cl_3$ | H | 5-$COOC_2H_5$ | 99-101 |
| 136 | " | " | 3-$COOC_2H_5$ | 114-115 |
| 137 | " | " | 5-COOH | |
| 138 | " | " | 3-COOH | |
| 139 | " | " | 5-$COOCH_3$ | |
| 140 | " | Br | 5-COOH | |
| 141 | " | Br | 3-COOH | |
| 142 | " | Cl | 5-COOH | |
| 143 | " | " | 3-COOH | |
| 144 | 4-$C_6H_5$ | H | 5-$COOC_2H_5$ | 40-43 |
| 145 | " | " | 3-$COOC_2H_5$ | 89-92 |
| 146 | " | " | 3-COOH | 196-199 |
| 147 | " | H | 5-$COOnC_{12}H_{25}$ | |
| 148 | " | Br | 5-COOH | |
| 149 | " | Br | 3-COOH | |
| 150 | " | Cl | 5-COOH | |
| 151 | " | " | 3-COOH | |
| 152 | 2-Cl | H | 5-$COOCH_3$ | 64-70 |
| 153 | " | " | 5-$COOC_2H_5$ | Öl |
| 154 | " | " | 5-COOH | 157-161 |
| 155 | " | " | 5-$CONH_2$ | |
| 156 | " | " | 5-$CONHC_2H_5$ | |
| 157 | " | " | 5-$CONHNHC_2H_5$ | |
| 158 | " | " | 5-$COSC_2H_5$ | |
| 159 | " | " | 5-COO-$nC_{12}H_{25}$ | |
| 160 | " | " | 3-$COOC_2H_5$ | |
| 161 | " | " | 3-$COSC_2H_5$ | |
| 162 | " | " | 3-COOH | |
| 163 | " | " | 3-$COOnC_4H_9$ | |
| 164 | " | Br | 5-$COOC_2H_5$ | Öl |

| Beispiel-Nr. | $R_n$ | 4-Y | X | Fp(°C) Kp (°C/torr) [°C/mbar] |
|---|---|---|---|---|
| 165 | 2-Cl | Br | $5-COSC_2H_5$ | |
| 166 | " | " | 5-COOH | |
| 167 | " | " | $3-COOC_2H_5$ | |
| 168 | " | Cl | $5-COOC_2H_5$ | |
| 169 | " | Cl | 5-COOH | |
| 170 | " | " | $3-COOC_2H_5$ | |
| 171 | " | " | $3-COSC_2H_5$ | |
| 172 | $2,4-Cl_2-5-OCH_3$ | H | $5-COOC_2H_5$ | Oel |
| 173 | " | " | 5-COOH | 187-190 |
| 174 | " | " | $3-COOC_2H_5$ | |
| 175 | " | Br | $5-COSC_2H_5$ | |
| 176 | " | Cl | $3-COOC_2H_5$ | |
| 177 | " | " | $5-COOC_2H_5$ | |
| 178 | $2,4-Cl_2-5-CO_2C_2H_5$ | H | $5-COOC_2H_5$ | (170-175/0,01) [170-175/0,013] |
| 179 | " | " | $5-COOCH_3$ | |
| 180 | " | " | $5-COO-c-C_6H_{11}$ | |
| 181 | " | " | $3-COOC_2H_5$ | |
| 182 | " | Br | $5-COOC_2H_5$ | |
| 183 | " | Cl | $5-COOC_2H_5$ | |
| 184 | $2-F-4-Cl-5-OCH_3$ | H | $5-COOC_2H_5$ | (155-162/0,01) [155-162/0,013] |
| 185 | " | " | 5-COOH | 207-210 |
| 186 | " | " | 5-CN | |
| 187 | " | " | $5-CONH_2$ | |
| 188 | " | " | $5-CNHNH_2$ | |
| 189 | " | " | $3-COOC_2H_5$ | |
| 190 | " | " | 3-COOH | |
| 191 | " | " | $5-COONH_4$ | |
| 192 | " | " | 5-COOK | |
| 193 | " | Cl | $5-COOCH_3$ | |
| 194 | " | Cl | 5-COOH | |
| 195 | " | " | $3-COOCH_3$ | |
| 196 | " | Br | $5-COOC_4H_9$ | |
| 197 | " | Br | $5-COOCH_2CCH$ | |

| Beispiel-Nr. | $R_n$ | 4-Y | X | Fp(°C) Kp (°C/Torr) [°C/mbar] |
|---|---|---|---|---|
| 198 | 2-F-4-Cl-5-$OCH_3$ | Br | 3-$COOC_2H_5$ | |
| 199 | 4-$CF_3$ | H | 5-$COOC_2H_5$ | 53-54 |
| 200 | " | " | 3-$COOC_2H_5$ | 79-84 |
| 201 | 4-$CF_3$-2,6-$(NO_2)_2$ | H | 5-$COOC_2H_5$ | 108-112 |
| 202 | " | " | 3-$COOC_2H_5$ | 138-142 |
| 203 | 2,Cl-4$CF_3$ | H | 5-$COOC_2H_5$ | 45-47 |
| 204 | " | " | 5-COOH | 149-150 |
| 205 | " | " | 3-$COOC_2H_5$ | 66-69 |
| 206 | 3-$CF_3$ | H | 5-$COOC_2H_6$ | (87-101/0,01) [87-101/0,013] |
| 207 | " | " | 3-$COOC_2H_5$ | 79-84 |
| 208 | " | " | 5-COOH | 136-138 |
| 209 | " | " | 3-$COO^-(Ca^{2+})_{1/2}$ | 244-261 |
| 210 | " | " | 3-COOK | 242 |
| 211 | " | " | 3-COONa | 283 |
| 212 | " | " | 5-$COO^-Ca^{2+}{}_{1/2}$ | 128-131 |
| 213 | " | " | 3-COO-c-$C_6H_{11}$ | 67-68 |
| 214 | " | Br | 3-COO-C-$C_6H_{11}$ | 86-91 |
| 215 | " | H | 5-COO-c-$C_6H_{11}$ | (155-160/0,5) [155-160/0,67] |
| 216 | " | Br | 5-COO-c-$C_6H_{11}$ | Oel |
| 217 | " | H | 5-$COO^-K^+$ | 208-213 |
| 218 | " | " | 5-$COO^-NH_4{}^+$ | 65-71 |
| 219 | " | " | 3-$COO^-NH_4{}^+$ | 207-212 |
| 220 | " | " | 3-$COO^-Li^+$ | >250 |
| 221 | " | " | 5-CONH-4-$C_6H_4$-4-Cl | |
| 222 | " | " | 5-$C(NH_2)NOCH_3$ | |
| 223 | " | " | 5-$COOCH_2CH_2$c-$C_6H_{11}$ | |
| 224 | " | " | 5-$CSOC_2H_5$ | |
| 225 | " | " | 3-$COSC_2H_5$ | |
| 226 | " | Br | 5-$COSC_2H_6$ | |
| 227 | " | Br | 3-$COSC_2H_6$ | |
| 228 | " | Cl | 5-$COONHCOCH_3$ | |
| 229 | " | Cl | 5-$COO(CH_2)_2OC_2H_2CH_3$ | |
| 230 | " | " | 5-$COOCH_2C_6H_5$ | |

| Beispiel-Nr | $R_n$ | 4-Y | X | Fp(°C) Kp (°C/Torr) [°C/mbar] |
|---|---|---|---|---|
| 231 | 2,4-$F_2$ | H | 5-$COOC_2H_5$ | (102-106/0,02 ) [102-106/0,027] |
| 232 | " | " | 3-$COOC_2H_5$ | (120-122/0,02) [120-122/0,027] |
| 233 | " | " | 5-COOH | 196-199 |
| 234 | " | Br | 5-COOH | 165-168 |
| 235 | " | Br | 3-$COOC_2H_5$ | |
| 236 | " | Cl | 5-COOH | |
| 237 | 4-F | H | 5-$COOC_2H_5$ | 96-98 |
| 238 | 4-F | H | 3-$COOC_2H_5$ | 44-49 |
| 239 | " | H | 5-COOH | 147-148 |
| 240 | " | H | 5-$COSC_2H_5$ | 62-65 |
| 241 | " | " | 5-$CSSC_2H_5$ | |
| 242 | " | " | 5-$CSN(CH_3)_2$ | |
| 243 | " | " | 5-$CONHNHCOC_6H_5$ | |
| 244 | " | " | 3-$COSC_2H_5$ | |
| 245 | " | " | 3-$CCNH_2$ | |
| 246 | " | Br | 5-COOH | 207 (Zers.) |
| 247 | " | Br | 5-CO-N 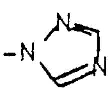 | |
| 248 | " | Br | 3-$COOC_2H_5$ | 79-83 |
| 249 | " | Cl | 5-COOH | |
| 250 | " | " | 3-COOH | |
| 251 | 4Br | H | 5-$COOC_2H_5$ | 63-65 |
| 252 | " | " | 5-$COOC_2H_5$ | 78-81 |
| 253 | " | " | 5-COOH | |
| 254 | " | " | 5-$COSC_2H_5$ | |
| 255 | " | " | 3-$COSC_2H_5$ | |
| 256 | " | Br | 5-COOH | |
| 257 | " | Cl | 5-COOH | |
| 258 | " | " | 3-COOH | |
| 259 | 4-Cl | H | 5-$COOC_2H_5$ | 60-65 |
| 260 | " | " | 3-COOH | 169-174 |

| Beispiel-Nr | $R_n$ | 4-Y | X | Fp(°C) |
|---|---|---|---|---|
| 261 | 4-Cl | H | 5-COOH | 181-182 |
| 262 | " | " | 3-$COOC_2H_5$ | 71-74 |
| 263 | " | Br | 3-$COOC_2H_5$ | 107-109 |
| 264 | " | " | 5-$COOC_2H_5$ | 109-112 |
| 265 | " | H | 5-$COO^-$ 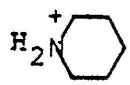 | 152-154 |
| 266 | " | " | 5-$COO^-$ 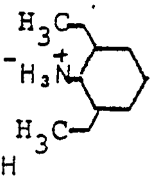 | Oel |
| 267 | " | Br | 5-COOH | 196-198 |
| 268 | " | " | 5-$COO^-HN^+(C_2H_4OH)_3$ | 112-114 |
| 269 | " | " | 5-$COO^-$ 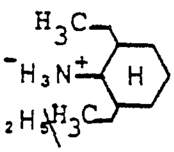 | Oel |
| 270 | 3-Cl | H | 5-$COOC_2H_5$ | 55-60 |
| 271 | " | " | 5-COOH | 205 |
| 272 | 3-Cl-5-$NO_2$ | H | 5-$COOC_2H_5$ | 104-116 |
| 273 | " | " | 3-$COOC_2H_5$ | 141-147 |
| 274 | 3-Cl | H | 3-COOH | |
| 275 | " | " | 3-$COSC_2H_5$ | |
| 276 | " | Br | 5-$COOCH_3$ | |
| 277 | " | Cl | 5-COOH | |
| 278 | " | " | 3-COOH | |
| 279 | 3-$COOC_2H_5$ | H | 3-$COOC_2H_5$ | 92-95 |
| 280 | " | " | 5-$COOC_2H_5$ | 85-87 |
| 281 | 3-$COO^-HN^+(C_2H_4OH)_3$ | H | 3-$COO^-HN^+(C_2H_4OH)_3$ | Oel |
| 282 | 3-COOH | H | 5-COOH | 236-238 |
| 283 | 3-COOH | H | 3-COOH | 240-243 |
| 284 | 4-COOH | H | 5-COOH | >260 |
| 285 | " | " | 3-COOH | >260 |
| 286 | 3-$OCF_2CHF_2$ | H | 5-COO-c-$C_6H_{11}$x$H_2SO_4$ | Oel |
| 287 | " | " | 5-$COOC_2H_5$ | Oel |
| 288 | " | H | 3-$COOC_2H_5$ | 47-51 |
| 289 | " | " | 5-COO-c-$C_6H_{11}$ | Oel |
| 290 | " | " | 3-COO-c-$C_6H_{11}$ | Oel |

| Beispiel-Nr | $R_n$ | 4-Y | X | Fp(°C) |
|---|---|---|---|---|
| 291 | $3-OCF_2CHF_2$ | H | 5-COO-i-Borneyl | Oel |
| 292 | " | " | 3-COO-i-Borneyl | 88-90 |
| 293 | " | Br | $5-COO-c-C_6H_{11}$ | Oel |
| 294 | " | " | $3-COOC_2H_5$ | 62-64 |
| 295 | " | " | $5-COOC_2H_5$ | Oel |
| 296 | " | Cl | $5-COOC_2H_5$ | |
| 297 | " | " | $3-COOC_2H_5$ | |
| 298 | $3-OCF_2CHFCF_3$ | H | $5-COOC_2H_5$ | Öl |
| 299 | " | " | 5-COOH | 129-131 |
| 300 | " | " | $5-COSC_2H_5$ | |
| 301 | " | H | 5-CN | |
| 302 | " | " | $3-COOC_2H_5$ | 44-46 |
| 303 | " | " | 3-COOH | 104 (Zers.) |
| 304 | " | H | $3-COSC_2H_5$ | |
| 305 | " | Br | $5-COOC_2H_5$ | |
| 306 | " | " | $3-COOC_2H_5$ | |
| 307 | $3-OCF_3$ | H | $5-COOC_2H_5$ | Öl |
| 308 | " | H | $3-COOC_2H_5$ | 55-58 |
| 309 | " | Cl | $5-COOC_2H_5$ | |
| 310 | $4-OCF_3$ | H | $5-COOC_2H_5$ | Öl |
| 311 | " | H | 5-COOH | 157-158 |
| 312 | " | " | $3-COOC_2H_5$ | 68-71 |
| 313 | " | Cl | $5-COOC_2H_5$ | 98-99 |
| 314 | $3-NO_2$ | H | $5-COOC_2H_5$ | 76-82 |
| 315 | $3-OCHF_2$ | H | $5-COOC_2H_5$ | |
| 316 | " | " | 5-COOH | |
| 317 | $2,4-F_2, 3,5-Cl_2$ | H | $5-COOC_2H_5$ | |
| 318 | " | " | $3-COOC_2H_5$ | |
| 319 | " | " | 5-COOH | |
| 320 | " | Br | $5-COOC_2H_5$ | |
| 321 | " | Cl | $3-COOC_2H_5$ | |
| 322 | $4-O-C_6H_5$ | H | $5-COOC_2H_5$ | |
| 323 | " | " | 5-COOH | |

| Beisp.-Nr. | $R_n$ | 4-Y | X | Fp(°C) |
|---|---|---|---|---|
| 324 | $4-O-C_6H_5$ | H | $3-COOC_2H_5$ | |
| 325 | $4-O-C_6H_4-2-Cl$ | H | $5-COOC_2H_5$ | |
| 326 | $4-NH_2$ | H | $3-COOC_2H_5$ | 84-87 |
| 327 | $3-NHCOCH_3$ | H | $5-COOC_2H_5$ | |
| 328 | 3-SH | H | $5-COOC_2H_5$ | |
| 329 | $3-S-C_6H_5$ | H | $5-COOC_2H_5$ | |
| 330 | $3-SO_2-C_6H_5$ | H | $5-COOCH_3$ | |
| 331 | $2,6-Cl_2-4-CF_3$ | H | $5-COOC_2H_5$ | 69-71 |
| 332 | " | H | $5-CONH_2$ | 171-173 |
| 333 | " | H | 5-CN | 67-69 |
| 334 | " | H | $3-COOC_2H_5$ | 112-115 |
| 335 | $4-NO_2$ | H | $3-COOC_2H_5$ | 159-161 |
| 336 | $3-C_2H_5$ | H | $5-COOC_2H_5$ | Oel |
| 337 | " | H | $3-COOC_2H_5$ | Oel |
| 338 | $3-OCF_3$ | H | 5-COOH | 113-115 |
| 339 | $4-OCF_3$ | Br | $3-COOC_2H_5$ | 92-97 |
| 340 | $4-F-3-NO_2$ | H | $5-COOC_2H_5$ | 74-76 |
| 341 | " | H | 5-COOH | 178 Zers. |
| 342 | $2,4,6-Cl_3$ | Br | $5-COOC_2H_5$ | 64-65 |
| 343 | $2,4,6-Cl_3-3-CH_3$ | H | $5-COOC_2H_5$ | 38-42 |
| 344 | 3-F- | H | $5-COOC_2H_5$ | Oel |
| 345 | 3-F | H | $3-COOC_2H_5$ | Oel |
| 346 | $2-CF_3$ | H | $5-COOC_2H_5$ | Oel |
| 347 | " | H | 5-COOH | 130-132 |
| 348 | $2-Cl-5-CF_3$ | H | $5-COOC_2H_5$ | Oel |
| 349 | " | H | $3-COOC_2H_5$ | Oel |

| Beisp.-Nr. | $R_n$ | 4-Y | X | Fp(°C) Kp |
|---|---|---|---|---|
| 350 | $3,5-(CF_3)_2$ | H | $5-COOC_2H_5$ | 63-67 |
| 351 | " | H | $3-COOC_2H_5$ | 108-110 |
| 352 | " | H | 5-COOH | 124-126 |
| 353 | $2,4-Cl_2-6-CH_3$ | H | $5-COOC_2H_5$ | 63-65 |
| 354 | $F_5$ | H | $5-COOC_2H_5$ | Oel |
| 355 | " | H | $3-COOC_2H_5$ | Oel |
| 356 | " | H | 5-COOH | 146-150 |
| 357 | $4-NHCH=C(CN)_2$ | H | $3-COOC_2H_5$ | >220 |
| 358 | 4- 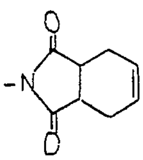 | H | $3-COOC_2H_5$ | 115-117 |
| 359 | $3-NHCOCOOC_2H_5$ | H | $5-COOC_2H_5$ | 50-54 |
| 360 | $2,4-Cl_2-5NO_2$ | Br | $5-CONH_2$ | 204-206 |
| 361 | $2,4,6-Cl_3-3NO_2$ | H | $5-COOC_2H_5$ | 94-101 |
| 362 | " | H | 5-COOH | 185-187 |
| 363 | " | H | 5-COOK | 189-192 |
| 364 | $3-CF_3$ | H | $5-CON(C_2H_5)_2$ | 66-68 |
| 365 | " | H | $5-CONHCH_2CH(OCH_3)_2$ | 92-94 |
| 366 | " | H | $5-CONH_2$ | 119-121 |
| 367 | " | H | $5-CONHCH_3$ | 72-77 |
| 368 | " | H | $5-CONHCH_2CH(C_2H_5)-n-C_4H_9$ | Oel |
| 369 | " | H | $5-CONH-c-C_6H_{11}$ | 134 Zers. |
| 370 | $2-Cl-4-CF_3$ | Br | $5-COOC_2H_5$ | Oel |
| 371 | " | Br | $3-COOC_2H_5$ | 38-41 |
| 372 | " | H | 5-COO- 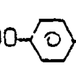 $-OCH(CH_3)COOC_2H_5$ | Oel |
| 373 | " | H | " COOH | 104-106 |

| Beips.-Nr. | $R_n$ | 4-Y | X | Fp(°C) |
|---|---|---|---|---|
| 374 | 2-Cl-5-$NO_2$ | H | 5-$COOC_2H_5$ | 78-82 |
| 375 | 2-Cl | H | 5-CO (Benzimidazol-1-yl) | 117-121 |
| 376 | " | H | 5-CON 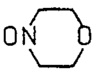 | 125-126 |
| 377 | 5-$NO_2$-2-$SC_6H_5$ | H | 5-$COOC_2H_5$ | Oel |
| 378 | 5-Cl-2-$NO_2$ | H | 5-$COOC_2H_5$ | 90-94 |
| 379 | 3-Cl-4-$NO_2$ | H | 5-$COOC_2H_5$ | 109-113 |
| 380 | 2,4-$(SC_6H_5)_2$-5-$NO_2$ | H | 5-$COOCH_3$ | 145-148 |
| 381 | 4-O-$CH_3$ | H | 5-$COOC_2H_5$ | Oel |
| 382 | " | H | 3-$COOC_2H_5$ | Oel |
| 383 | " | H | 5-COOH | 170-172 |
| 384 | " | H | 3-COOH | 185-187 |
| 385 | 2,3,5,6-$F_4$ | H | 5-$COOC_2H_5$ | 57-60 |
| 386 | " | H | 5-COOH | 128-130 |
| 387 | " | H | 5-$CON(C_2H_5)_2$ | 80-83 |
| 388 | " | H | 5-COO-n-$C_6H_{13}$ | Oel |
| 389 | 3- 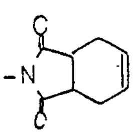 | H | 5-$COOC_2H_5$ | 96-101 |
| 390 | 3-$NO_2$-4-$OC_6H_5$ | H | 5-$COOC_2H_5$ | 52-54 |
| 391 | " | H | 5-COOH | 178-181 |
| 392 | 4-NH-$SO_2CH_3$ | H | 3-$COOC_2H_5$ | 150-155 |
| 393 | 3-Cl-4-F | H | 5-$COOC_2H_5$ | 84-87 |
| 394 | " | H | 3-$COOC_2H_5$ | 122-125 |
| 395 | " | H | 5-COOH | >225 |
| 396 | 4-F-3-$CF_3$ | " | 3-$COOC_2H_5$ | 24-29 |
| 397 | 4-$N(CH_3)_2$-3-$CF_3$ | H | 5-$COOC_2H_5$ | Oel |
| 398 | " | H | 3-$COOC_2H_5$ | Oel |

| Beisp.-Nr. | $R_n$ | 4-Y | X | Fp(°C) |
|---|---|---|---|---|
| 399 | 3-Cl-2,6-($C_2H_5$) | H | 5-$COOC_2H_5$ | Oel |
| 400 | " | H | 3-$COOC_2H_5$ | Oel |
| 401 | " | H | 5-COOH | 145-147 |
| 402 | " | Br | 5-$COOC_2H_5$ | Oel |
| 403 | 2,4-$Br_2$ | H | 5-$COOC_2H_5$ | Oel |
| 404 | " | H | 3-$COOC_2H_5$ | 103-105 |
| 405 | " | H | 5-COOH | 217-219 |
| 406 | " | Br | 5-$COOC_2H_5$ | Oel |
| 407 | 2,4-$Cl_2$ | H | 3-$CONHSO_2CH_3$ | 155-159 |
| 408 | " | H | 3-$COOCH_3$ | 105-107 |
| 409 | " | H | 3-$COOCH_2C{\equiv}CH$ | 101-103 |
| 410 | " | H | 5-$COOCH_2C{\equiv}CH$ | Oel |
| 411 | " | H | 5-$COOCH(CH_3)_2$ | Oel |
| 412 | " | H | 5-$COOCH_2CCl_3$ | Oel |
| 413 | " | H | 5-$COONC(CH_3)_2$ | 87-89 |
| 414 | " | H | 5-$COOCH(CF_3)_2$ | Oel |
| 415 | " | H | 5-CN | 70-71 |
| 416 | " | H | 5-$COOCH_2Si(CH_3)_3$ | Oel |
| 417 | " | H | 3-$COOCH_2Si(CH_3)_3$ | 51-54 |
| 418 | " | H | 5-CON 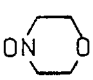 | Oel |

Biologische Beispiele

A. Wachstumsregulierung

1. Wuchshemmung an Getreide

In Schalenversuchen im Gewächshaus wurden junge Getreidepflanzen (Weizen, Gerste, Roggen) im 3-Blattstadium mit erfindungsgemässen Verbindungen in verschiedenen Wirkstoffkonzentrationen (kg/ha)

tropfnass gespritzt.

Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet, wobei 100% den Stillstand des Wachstums und 0% ein Wachstum entsprechend den unbehandelten Kontrollpflanzen bedeuten. Es zeigte sich, daß die Verbindungen sehr gute wachstumsregulierende Eigenschaften besitzen. Die Ergebnisse sind in der nachfolgenden Tabelle zusammengestellt.

Tabelle

| Verbindungen nach Bsp.Nr. | Anwendungs- konz. kg/ha | Wuchshemmung (%) Weizen | Gerste | Roggen | Phytotox. Wirkung |
|---|---|---|---|---|---|
| 17 | 2.5 | 15 | 22 | 19 | keine |
| | 1.25 | 11 | 16 | 14 | Schäden |
| 34 | " | 14 | 21 | 17 | keine |
| | " | 10 | 14 | 11 | Schäden |
| 42 | " | 25 | 38 | 22 | keine |
| | " | 22 | 23 | 17 | Schäden |
| 43 | " | 24 | 38 | 23 | keine |
| | " | 21 | 22 | 16 | Schäden |
| 44 | " | 24 | 37 | 23 | keine |
| | " | 20 | 23 | 17 | Schäden |
| 52 | " | 22 | 31 | 21 | keine |
| | " | 18 | 26 | 17 | Schäden |
| 53 | " | 16 | 21 | 19 | keine |
| | " | 10 | 15 | 13 | Schäden |
| 55 | " | 14 | 20 | 21 | keine |
| | " | 9 | 13 | 14 | Schäden |
| 62 | " | 18 | 21 | 14 | keine |
| | " | 14 | 15 | 12 | Schäden |
| 72 | " | 14 | 17 | 14 | keine |
| | " | 12 | 15 | 9 | Schäden |
| 83 | " | 19 | 22 | 19 | keine |
| | " | 12 | 14 | 13 | Schäden |
| 88 | " | 23 | 36 | 29 | keine |
| | " | 18 | 28 | 20 | Schäden |
| 89 | " | 26 | 39 | 24 | keine |
| | " | 21 | 24 | 19 | Schäden |
| 90 | " | 14 | 21 | 18 | keine |
| | " | 10 | 16 | 13 | Schäden |
| 92 | " | 17 | 22 | 19 | keine |
| | " | 11 | 17 | 14 | Schäden |

| Verbindungen nach Bsp.Nr. | Anwendungs-konz. kg/ha | Wuchshemmung (%) Weizen | Gerste | Roggen | Phytotox. Wirkung |
|---|---|---|---|---|---|
| 115 | 2.5 | 16 | 21 | 19 | keine |
| | 1.25 | 11 | 17 | 14 | Schäden |
| 116 | " | 17 | 22 | 19 | keine |
| | " | 12 | 17 | 13 | Schäden |
| 117 | " | 19 | 24 | 21 | keine |
| | " | 14 | 19 | 16 | Schäden |
| 128 | " | 16 | 21 | 17 | keine |
| | " | 11 | 16 | 13 | Schäden |
| 129 | " | 22 | 31 | 22 | keine |
| | " | 18 | 25 | 19 | Schäden |
| 135 | " | 15 | 19 | 18 | keine |
| | " | 11 | 16 | 14 | Schäden |
| 140 | " | 20 | 24 | 22 | keine |
| | " | 14 | 19 | 17 | Schäden |
| 153 | " | 20 | 23 | 21 | keine |
| | " | 13 | 19 | 16 | Schäden |
| 154 | " | 22 | 27 | 24 | keine |
| | " | 15 | 23 | 19 | Schäden |
| 178 | " | 14 | 19 | 19 | keine |
| | " | 12 | 14 | 15 | Schäden |
| 185 | " | 13 | 18 | 15 | keine |
| | " | 9 | 13 | 9 | Schäden |
| 204 | " | 16 | 19 | 17 | keine |
| | " | 11 | 16 | 15 | Schäden |
| 206 | " | 15 | 20 | 18 | keine |
| | " | 13 | 13 | 14 | Schäden |
| 208 | " | 20 | 35 | 22 | keine |
| | " | 14 | 24 | 17 | Schäden |
| 217 | " | 17 | 27 | 22 | keine |
| | " | 14 | 22 | 17 | Schäden |
| 218 | " | 18 | 27 | 19 | keine |
| | " | 15 | 23 | 16 | Schäden |
| 246 | " | 25 | 38 | 27 | keine |
| | " | 21 | 29 | 24 | Schäden |
| 267 | " | 21 | 30 | 22 | keine |
| | " | 17 | 23 | 17 | Schäden |
| 269 | " | 24 | 37 | 27 | keine |
| | " | 21 | 28 | 23 | Schäden |

| Verbindungen nach Bsp.Nr. | Anwendungs-konz. kg/ha | Wuchshemmung (%) Weizen | Gerste | Roggen | Phytotox. Wirkung |
|---|---|---|---|---|---|
| 295 | 2.5 | 19 | 29 | 22 | keine |
| | 1.25 | 16 | 24 | 17 | Schäden |
| 356 | " | 19 | 28 | 21 | keine |
| | " | 15 | 22 | 16 | Schäden |
| 366 | " | 17 | 21 | 17 | keine |
| | " | 11 | 16 | 13 | Schäden |
| 405 | " | 24 | 37 | 23 | keine |
| | " | 21 | 28 | 18 | Schäden |
| 413 | " | 19 | 26 | 18 | keine |
| | " | 13 | 19 | 13 | Schäden |

2. Wuchshemmung in Wasserreis

Reispflanzen wurden in Töpfen im Gewächshaus biz zum 3-Blattstadium angezogen, und dann mit den erfindungsgemässen Verbindungen behandelt. Die Substanzen wurden sowohl durch Spritzung appliziert als auch in das Wasser gegeben.

3 Wochen nach Behandlung wurde bei allen Pflanzen der Zuwachs gemessen und die Wuchshemmung in % des Zuwachses der Kontrollpflanzen berechnet. Es wurde außerdem auf eine mögliche phytotoxische Wirkung der Verbindungen geachtet.

Die Wuchshemmung wurde als prozentualer Wert ermittelt, wobei 100% den Stillstand des Wachstums und 0% ein Wachstum entsprechend dem der unbehandelten Kontrollpflanzen bedeuten.

Die Ergebnisse sind in der nachfolgenden Tabelle zusammengefaßt.

Tabelle

| Verbindungen nach Bsp. Nr. | Anwendungs-konz. kg/ha | Wuchshemmung (%) | Phytotox. Wirkung |
|---|---|---|---|
| 42 | 2.5 | 26 | keine |
| | 1.25 | 24 | Schäden |
| | 0.62 | 20 | |
| 43 | " | 27 | keine |
| | " | 24 | Schäden |
| | " | 19 | |
| 62 | " | 19 | keine |
| | " | 15 | Schäden |
| | " | 8 | |
| 83 | " | 21 | keine |
| | " | 16 | Schäden |
| | " | 13 | |
| 88 | " | 19 | keine |
| | " | 16 | Schäden |
| | " | 12 | |
| 178 | " | 22 | keine |
| | " | 17 | Schäden |
| | " | 15 | |
| 206 | " | 25 | keine |
| | " | 19 | Schäden |
| | " | 17 | |
| 208 | " | 32 | keine |
| | " | 27 | Schäden |
| | " | 21 | |
| 218 | " | 26 | keine |
| | " | 20 | Schäden |
| | " | 17 | |
| 219 | " | 27 | keine |
| | " | 21 | Schäden |
| | " | 17 | |
| 246 | " | 29 | keine |
| | " | 25 | Schäden |
| | " | 21 | |

3. Wuchshemmung an Sojabohnen

Ca. 10 cm große Sojabohnen wurden mit den Wirkstoffzubereitungen tropfnaß bespritzt. Nach 3 Wochen wurde bonitiert.

Die Wuchshemmung wurde als prozentualer Wert ermittelt, wobei 100 % den Stillstand des Wachstums und 0 % ein Wachstum entsprechend dem der unbehandelten Kontrollpflanzen bedeutet.

Tabelle

| Verbindungen nach Bsp.Nr. | Anwendungs-konz. kg/ha | Wuchshemmung (%) | Phytotox. Wirkung |
|---|---|---|---|
| 35 | 2.5 | 22 | keine Schäden |
| 88 | 2.5 | 25 | " |
| 89 | 2.5 | 27 | " |
| 42 | 2.5 | 26 | " |
| 43 | 2.5 | 24 | " |
| 44 | 2.5 | 26 | " |

B. Safener - Wirkung

Beispiel 1

Getreide, vorzugsweise Weizen, wurde im Gewächshaus in Plastiktöpfen von 9 cm Durchmesser biz zum 3-4 Blattstadium herangezogen und dann gleichzeitig mit den erfindungsgemäßen Verbindungen und den getesteten Herbiziden im Nachlaufverfahren behandelt. Herbizide und die Verbindungen der Formel I wurden dabei in Form wässriger Suspensionen bzw. Emulsionen mit einer Wasseraufwandmenge von umgerechnet 800 1/ha ausgebracht. 3-4 Wochen nach der Behandlung wurde die Pflanzen visuell auf jede Art von Schädigung durch die ausgebrachten Herbizide bonitiert, wobei insbesondere das Ausmaß der anhaltenden Wachstumshemmung berücksichtigt wurde.

Die Ergebnisse aus Tabelle V veranschaulichen, daß die erfindungsgemäßen Verbindungen starke Herbizidschäden an den Kulturpflanzen effektiv reduzieren können.

Selbst bei starken Überdosierungen des Herbizids werden bei den Kulturpflanzen auftretende schwere Schädigungen deutlich reduziert, geringere Schäden völlig aufgehoben. Mischungen aus Herbiziden und erfindungsgemäßen Verbindungen eignen sich deshalb in ausgezeichneter Weise zur selektiven Unkrautbekämpfung in Getreidekulturen.

Beispiel 2

Getreide und die beiden Schadgräser Avena fatua und Alopecurus myosuroides wurden in Plastiktöpfen von 9 bzw. 13 cm Durchmesser in lehmigen Sandboden ausgesät, unter optimalen Wuchsbedingungen im Gewächshaus bis zum 3-4 Blattstadium bzw. zur beginnenden Bestockung angezogen und mit Mischungen aus den erfindungsgemäßen Verbindungen und den Herbiziden behandelt. Die Präparate wurden dabei in Form wässriger Suspensionen oder Emulsionen mit einer Wasseraufwandmenge von umgerechnet 300 - 600 l/ha ausgebracht.

3-4 Wochen nach der Applikation wurden die Versuchspflanzen auf Wachstumsveränderungen und Schädigung im Vergleich zu unbehandelten und mit den Herbiziden alleine behandelten Kontrollen visuell bonitiert.

Die Ergebnisse aus der Tabelle V zeigen, daß die erfingsgemäßen Verbindungen sehr gute Safenereigenschaften bei Getreidepflanzen aufweisen und somit Herbizidschäden wirkungsvoll verhindern können, ohne die eigentliche herbizide Wirkung gegen Schadgräser zu beeinträchtigen.

Mischungen aus Herbiziden und erfindungsgemäßen Verbindungen können somit zur selektiven Unkrautbekämpfung eingesetzt werden.

Safenerwirkung der erfindungsgemäßen Verbindungen. Schädigung der Kulturpflanzen in %.

Tabelle

| Beispiel-Nr. | herbizide Wirkung TA | HV |
|---|---|---|
| $H_1$ | 85 | 80 |
| $H_1$ + 16 | 40 | - |
| $H_1$ + 17 | 45 | - |
| $H_1$ + 26 | 40 | - |
| $H_1$ + 27 | 40 | - |
| $H_1$ + 30 | 50 | - |
| $H_1$ + 34 | 40 | - |
| $H_1$ + 45 | 20 | 35 |
| $H_1$ + 46 | 30 | 40 |
| $H_1$ + 47 | 30 | - |
| $H_1$ + 48 | 30 | - |
| $H_1$ + 49 | - | 50 |
| $H_1$ + 50 | 30 | - |
| $H_1$ + 51 | - | 50 |
| $H_1$ + 54 | - | 50 |
| $H_1$ + 65 | 30 | - |
| $H_1$ + 84 | 40 | 55 |
| $H_1$ + 96 | 30 | - |
| $H_1$ + 98 | 50 | - |
| $H_1$ + 99 | - | 40 |
| $H_1$ + 128 | - | 50 |
| $H_1$ + 136 | 20 | - |
| $H_1$ + 153 | 30 | 65 |
| $H_1$ + 154 | 40 | - |
| $H_1$ + 164 | 40 | - |
| $H_1$ + 178 | 50 | - |
| $H_1$ + 201 | 30 | - |
| $H_1$ + 204 | 40 | 35 |

| Beispiel-Nr. | herbizide Wirkung TA | HV |
|---|---|---|
| $H_1$ + 205 | 50 | 30 |
| $H_1$ + 209 | 50 | - |
| $H_1$ + 210 | 35 | - |
| $H_1$ + 211 | 40 | 55 |
| $H_1$ + 218 | - | 40 |
| $H_1$ + 219 | 35 | - |
| $H_1$ + 220 | 50 | - |
| $H_1$ + 237 | 40 | - |
| $H_1$ + 238 | 30 | - |
| $H_1$ + 239 | 50 | - |
| $H_1$ + 240 | 50 | - |
| $H_1$ + 246 | 40 | 30 |
| $H_1$ + 251 | 30 | - |
| $H_1$ + 252 | 30 | 40 |
| $H_1$ + 259 | 30 | - |
| $H_1$ + 260 | 40 | 50 |
| $H_1$ + 261 | 50 | 40 |
| $H_1$ + 262 | 40 | 45 |
| $H_1$ + 265 | - | 50 |
| $H_1$ + 269 | - | 50 |
| $H_1$ + 270 | 60 | 50 |
| $H_1$ + 271 | 20 | 45 |
| $H_1$ + 279 | 50 | - |
| $H_1$ + 280 | 50 | - |
| $H_1$ + 286 | 10 | 40 |
| $H_1$ + 288 | 30 | 40 |
| $H_1$ + 289 | 40 | - |
| $H_1$ + 293 | 50 | - |
| $H_1$ + 294 | 40 | - |
| $H_1$ + 295 | 50 | - |
| $H_1$ + 298 | - | 50 |
| $H_1$ + 311 | 40 | 40 |
| $H_1$ + 312 | 40 | 50 |
| $H_1$ + 314 | 40 | - |

| Beispiel-Nr. | herbizide Wirkung TA | HV |
|---|---|---|
| $H_1$ + 331 | 40 | - |
| $H_1$ + 334 | 20 | 50 |
| $H_1$ + 340 | 40 | - |
| $H_1$ + 342 | 40 | - |
| $H_1$ + 343 | 40 | - |
| $H_1$ + 344 | 40 | - |
| $H_1$ + 346 | 40 | - |
| $H_1$ + 347 | 40 | - |
| $H_1$ + 348 | 30 | - |
| $H_1$ + 349 | 20 | 50 |
| $H_1$ + 350 | 40 | 50 |
| $H_1$ + 352 | - | 50 |
| $H_1$ + 353 | 40 | - |
| $H_1$ + 371 | 40 | 35 |
| $H_1$ + 373 | 45 | 60 |
| $H_1$ + 375 | 35 | - |
| $H_1$ + 389 | 20 | 50 |
| $H_1$ + 391 | 40 | - |
| $H_1$ + 394 | 40 | - |
| $H_1$ + 395 | 40 | - |
| $H_1$ + 407 | 40 | 35 |
| $H_1$ + 408 | 35 | 35 |
| $H_1$ + 409 | 40 | 40 |
| $H_1$ + 410 | 60 | 50 |
| $H_1$ + 415 | 40 | - |
| $H_1$ + 416 | 30 | 60 |
| $H_1$ + 417 | 40 | 40 |

Erklärungen und Abkürzungen

Dosierungen der Mischungspartner:

$H_1$ : 2,0 kg a.i. / ha (TA)
0,3 kg a.i. / ha (HV)
Safener : 2,5 kg a.i. / ha
$H_1$ = Fenoxaprop - ethyl
TA = Triticum aestivum
HV = Hordeum vulgare

**Ansprüche**

1. Verbindungen der Formel I, deren Salze und Quaternisierungsprodukte,

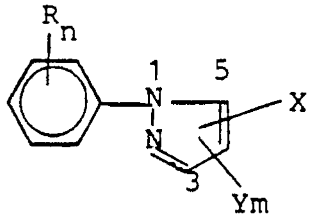 (I),

worin

R unabhängig voneinander Halogen, Hydroxy, Cyano, Nitro, $(C_1-C_4)$Alkyl, $(C_1-C_4)$Halogenalkyl, $(C_1-C_4)$Alkoxy-$(C_1-C_4)$alkyl, $(C_1-C_6)$Alkoxy, $(C_1-C_6)$Alkoxy-$(C_1-C_4)$alkoxy, $(C_1-C_6)$Halogenalkoxy, $(C_1-C_4)$-Alkylthio, $(C_1-C_4)$Halogenalkylthio, Carboxy, $(C_1-C_4)$Alkoxycarbonyl, $(C_1-C_4)$Alkylsulfinyl, $(C_1-C_4)$-Halogenalkylsulfinyl, $(C_1-C_4)$Alkylsulfonyl, $(C_1-C_4)$Halogenalkylsulfonyl, $(C_1-C_4)$-Alkylsulfonyloxy, $(C_1-C_4)$Halogenalkylsulfonyloxy, Phenyl, Halogenphenyl, Phenoxy oder Halogenphenoxy,

X = in Position 3 oder 5 des Pyrazolringes orientiert ist und einen Rest der Formeln

-CN, 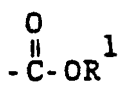, 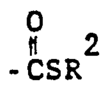, 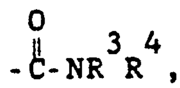,

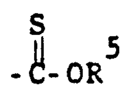, 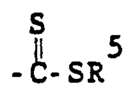, 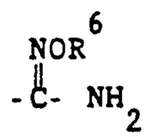, 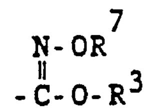

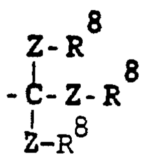, 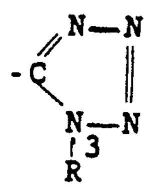, 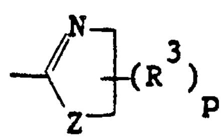, 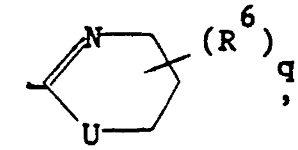,

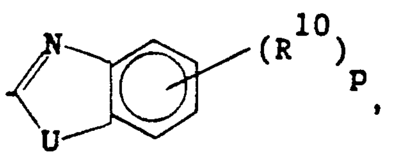, 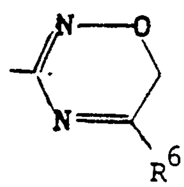 oder 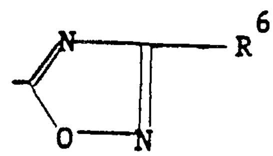

Y = Halogen

Z = O oder S

U = O, S oder N-$R^6$.

$R^1$ Wasserstoff, $(C_1-C_{12})$Alkyl, $(C_1-C_{12})$Alkyl, das ein- oder mehrfach durch Halogen und/oder ein- bis zweifach durch Hydroxy, $(C_1-C_6)$Alkoxy, $(C_1-C_4)$Alkoxy$(C_1-C_4)$alkoxy, $(C_1-C_4)$-Alkylthio, $(C_1-C_4)$-Alkylsulfinyl, $(C_1-C_4)$Alkylsulfonyl, Mono- oder Di-$(C_1-C_4$-alkyl)amino, Cyano, Aminocarbonyl, $(C_1-C_4)$Alkylcarbonyl, $(C_1-C_4$-Alkoxy)carbonyl, Cyclo$(C_3-C_7)$-alkyl, Tri$(C_1-C_4)$alkyl-silyl, Benzyloxy, Benzyloxyethoxy, Phenyl, Phenyl, das durch Halogen oder $(C_1-C_4)$Alkyl substituiert ist, durch Phenoxy, Phenylthio, die durch Halogen oder $(C_1-C_4)$-Alkyl substituiert sein können, durch Oxiranyl, Tetrahydrofuryl, Triazolyl, Pyridinyl, Imidazolyl, durch Carboxy, Carboxylat mit einem für die Landwirtschaft einsetzbaren Kation oder durch den Rest -O-N=C$(CH_3)_2$ substituiert ist, $(C_3-C_6)$Alkenyl, $(C_3-C_6)$-Halogenalkenyl, unsubstituiertes oder durch Halogen oder $(C_1-C_4)$Alkyl substituiertes Cyclo$(C_3-C_7)$-alkyl, unsubstituiertes oder durch Halogen oder $(C_1-C_4)$Alkyl substituiertes Cyclo$(C_5-C_7)$alkenyl, $(C_3-C_6)$Alkinyl, 1,2-Epoxy-prop-3-yl, Phenyl oder Phenyl, das ein oder zweifach durch Halogen, Nitro, Cyano, $(C_1-C_4)$Alkyl, $(C_1-C_4$-Alkoxy)carbonyl oder $(C_1-C_4)$Alkoxy substituiert ist, $(C_1-C_4$-Alkyl)-

carbonyl, Phenylcarbonyl, wobei der Phenylring durch Halogen, Nitro, Cyano oder $(C_1-C_4)$Alkyl substituiert sein kann, einen Rest der Formeln

$-N=C(R^{10})_2$ , $-NR^3R^{11}$, 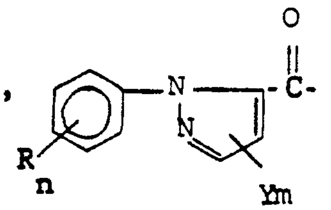 , 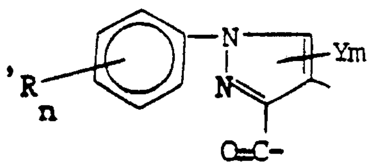

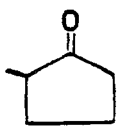 , 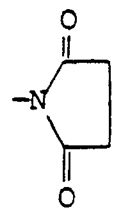 , 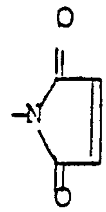 , 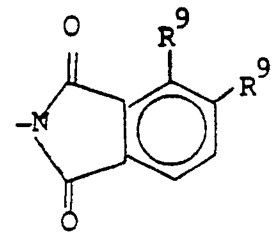

oder ein für die Landwirtschaft einsetzbares Kation,
$R^2$ $(C_1-C_{12})$Alkyl oder $(C_1-C_{12})$Alkyl, das bis zu zweifach durch $(C_1-C_4)$Alkoxyethoxy , Cyclo$(C_3-C_6)$-alkyl, Benzyloxy, Phenyl, Phenoxy, $(C_1-C_4)$Alkylthio, $(C_1-C_4$-Alkoxy)-carbonyl, Carboxy oder Carboxylat mit einem für die Landwirtschaft einsetzbares Kation, substituiert ist,
$R^3$ jeweils unabhängig voneinander $(C_1-C_6)$-Alkyl, Phenyl oder $(C_3-C_6)$-Alkenyl,
$R^4$ Wasserstoff, $(C_1-C_{12})$Alkyl oder $(C_1-C_{12})$Alkyl, das bis zu zweifach durch $(C_1-C_6)$Alkoxy, $(C_1-C_4)$-Alkoxy-ethoxy, Hydroxy, Hydroxyimino, $(C_1-C_4)$-Alkoxyimino, Halogen, Cyclo$(C_3-C_6)$alkyl, Benzyloxy, Cyano, Aminocarbonyl, Carboxy, $(C_1-C_4$-Alkoxy)-carbonyl, Formyl, Phenyl oder Phenoxy substituiert ist, Phenyl oder Phenyl, das bis zu zweifach durch Halogen, Nitro, Cyano, $(C_1-C_4)$Alkyl oder $(C_1-C_4)$-Alkoxy substituiert ist; $(C_3-C_6)$-Alkenyl, $(C_3-C_6)$Cycloalkyl, einen Rest der Formeln $-NR^3R^{12}$, $-O-R^6$, $-NH-CONH_2$, $-NH-CS-NH_2$ oder $-SO_2R^{13}$ oder
$R^3$ und $R^4$ gemeinsam mit dem Stickstoffatom an das sie gebunden sind, einen gesättigten oder ungesättigten gegebenenfalls benzokonensierten drei- bis siebengliedrigen Ring, der bis zu drei Heteroatome aus der Gruppe O, N oder S enthält und der unsubstituiert oder durch $(C_1-C_4)$Alkyl oder Halogen substituert ist und eine Carbonylgruppe enthalten kann,
$R^5$ H, $(C_1-C_6)$Alkyl oder Phenyl, oder im Falle X = $-CS-OR^5$ ein für die Landwirtschaft einsetzbares Kation,
$R^6$ jeweils unabhängig voneinander H, $(C_1-C_4)$Alkyl oder Benzyl,
$R^7$ jeweils unabhängig voneinander H, $(C_1-C_{12})$Alkyl, das unsubstituiert oder durch Phenyl, das unsubstituiert oder durch Halogen, Nitro, Cyano, $(C_1-C_4)$Alkyl oder $(C_1-C_4)$Alkoxy substituiert ist, durch Hydroxy, Cyano, $(C_1-C_4$-Alkoxy)-carbonyl, $(C_1-C_4)$-Alkylthio, $(C_1-C_4)$-Alkoxy, Cyclo$(C_5-C_7)$alkyl oder Benzyloxy substituiert ist,
$(C_3-C_6)$Alkenyl, Halogen$(C_3-C_6)$Alkenyl, $(C_3-C_6)$Alkinyl, Cyclo$(C_5-C_8)$alkyl, Cyclo$(C_5-C_6)$alkenyl, $(C_1-C_6$-Alkyl)carbonyl, Halogen$(C_1-C_6$-alkyl)carbonyl, $[(C_1-C_6$-Alkyl)amino]carbonyl, Benzoyl, Halogenbenzoyl oder Methylbenzoyl
$R^8$ jeweils unabhängig voneinander $(C_1-C_6)$Alkyl, das unsubstituiertes oder durch Phenyl, Cyclo$(C_5-C_7)$alky, $(C_1-C_4)$Alkoxy, $(C_1-C_4)$Alkylthio oder Halogen substituiert ist, oder zwei Reste $R^8$ gemeinsam mit Z und dem Kohlenstoffatom, an das sie gebunden sind, einen unsubstituierten oder durch $(C_1-C_4)$Alkyl, Hydroxy$(C_1-C_4)$alkyl, Halogen$(C_1-C_4)$alkyl oder Phenyl substituierten 5- oder 6-gliedrigen gesättigten heterocyclischen Ring;
$R^9$ jeweils unabhängig voneinander H, Halogen, $(C_1-C_4)$Alkyl, Nitro oder Cyano,
$R^{10}$ unabhängig voneinander H, $(C_1-C_6)$Alkyl, das unsubstituiert oder durch $(C_1-C_4)$Alkoxy, Triazolyl oder Imidazolyl substituiert ist, Cyclo$(C_3-C_6)$alkyl, $(C_3-C_6)$Alkenyl, Phenyl oder Benzyl, oder im Falle $R^1$ $=-N=C(R^{10})_2$ beide Reste $R^{10}$ gemeinsam mit dem Kohlenstoffatom, an das sie gebunden sind, ein unsubstituiertes oder durch Methyl oder Halogen substituiertes Cyclo-$(C_5-C_7)$alkyl,
$R^{11}$ $(C_1-C_4)$Alkyl, Phenyl, $(C_1-C_6$-Alkyl)carbonyl, Benzyl, Benzoyl, Halogenbenzyl, Halogenbenzoyl oder Methylbenzoyl,
$R^{12}$ H, $(C_1-C_4)$Alkyl, Formyl, $(C_1-C_6$-Alkyl)carbonyl, Benzoyl, Halogenbenzoyl, Methylbenzoyl oder Trihalogenacetyl,

$R^{13}$ ($C_1$-$C_4$)Alkyl, Phenyl oder Methylphenyl,
m 0 oder 1,
n eine ganze Zahl von 0 bis 5,
p eine ganze Zahl von 0 bis 4 und
q eine ganze Zahl von 0 bis 6 bedeuten.

**2.** Verfahren zur Herstellung der Verbindungen der Formel I von Anspruch 1, dadurch gekennzeichnet, daß man eine Verbindung der Formel II

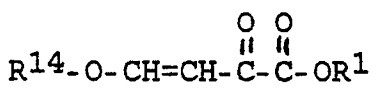

(II)

worin $R^{14}$ ($C_1$-$C_6$)Alkyl bedeutet, mit einer Verbindung der Formel III

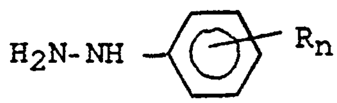

(III)

umsetzt und anschließend gegebenenfalls derivatisiert.

**3.** Pflanzenbehandlungsmittel, gekennzeichnet durch einen wirksamen Gehalt einer Verbindung der Formel I von Anspruch 1.

**4.** Pflanzenwachstumsregulierende Mittel, gekennzeichnet durch einen wirksamen Gehalt einer Verbindung der Formel I von Anspruch 1.

**5.** Mittel zum Schutz von Kulturpflanzen vor phytotoxischen Nebenwirkungen von Herbiziden, gekennzeichnet durch einen wirksamen Gehalt einer Verbindung der Formel I von Anspruch 1.

**6.** Verwendung der Verbindungen der Formel I zur Wachstumsregulierung von Pflanzen.

**7.** Verwendung der Verbindungen der Formel I zum Schutz von Kulturpflanzen vor phytotoxischen Nebenwirkungen von Herbiziden.

**8.** Verfahren zur Regulierung des Pflanzenwachstums, dadurch gekennzeichnet, daß man auf die Pflanzen oder die Anbaufläche eine wirksame Menge einer Verbindung der Formel I appliziert.

**9.** Verfahren zum Schutz von Kulturpflanzen vor phytotoxischen Nebenwirkungen von Herbiziden, dadurch gekennzeichnet, daß man auf die Kulturpflanzen oder die Anbaufläche eine wirksame Menge einer Verbindund der Formel I vor, nach oder gleichzeitig mit dem Herbizid appliziert.

**10.** Verfahren gemäß Anspruch 9, dadurch gekennzeichnet, daß das Herbizid ein Phenoxy-phenoxy- oder Heteroaryloxyphenoxy-carbonsäureester ist.

Patentansprüche für folgende Vertragsstaaten : AT, ES

**1.** Verfahren zur Herstellung von Verbindungen der Formel I

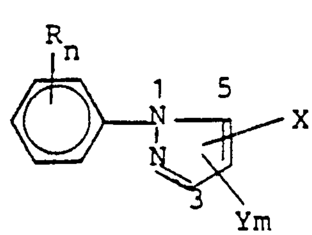

(I),

worin

R unabhängig voneinander Halogen, Hydroxy, Cyano, Nitro, $(C_1\text{-}C_4)$Alkyl, $(C_1\text{-}C_4)$Halogenalkyl, $(C_1\text{-}C_4)$Alkoxy-$(C_1\text{-}C_4)$alkyl, $(C_1\text{-}C_6)$Alkoxy, $(C_1\text{-}C_6)$Alkoxy-$(C_1\text{-}C_4)$alkoxy, $(C_1\text{-}C_6)$Halogenalkoxy, $(C_1\text{-}C_4)$-Alkylthio, $(C_1\text{-}C_4)$Halogenalkylthio, Carboxy, $(C_1\text{-}C_4)$Alkoxycarbonyl, $(C_1\text{-}C_4)$Alkylsulfinyl, $(C_1\text{-}C_4)$-Halogenalkylsulfinyl, $(C_1\text{-}C_4)$Alkylsulfonyl, $(C_1\text{-}C_4)$Halogenalkylsulfonyl, $(C_1\text{-}C_4)$-Alkylsulfonyloxy, $(C_1\text{-}C_4)$Halogenalkylsulfonyloxy, Phenyl, Halogenphenyl, Phenoxy oder Halogenphenoxy,

X = in Position 3 oder 5 des Pyrazolringes orientiert ist und einen Rest der Formeln

-CN, 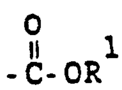, 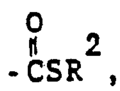, 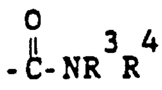,

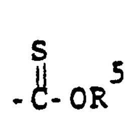, 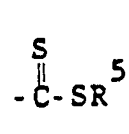, 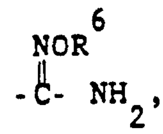, 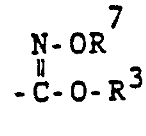

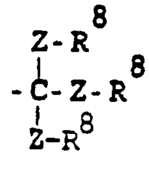, 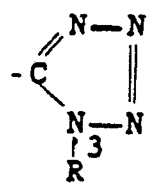, 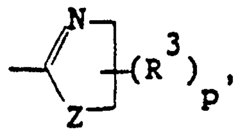, 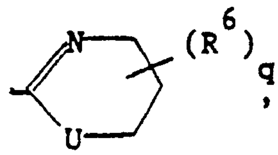,

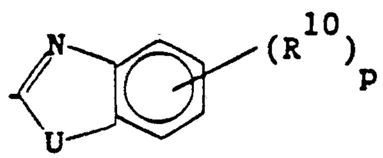, 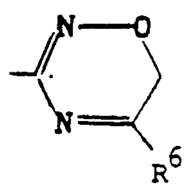 oder 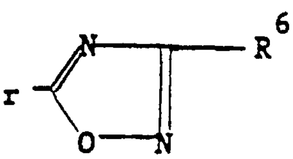

Y = Halogen

Z = O oder S

U = O, S oder N-$R^6$.

$R^1$ Wasserstoff, $(C_1\text{-}C_{12})$Alkyl, $(C_1\text{-}C_{12})$Alkyl, das ein- oder mehrfach durch Halogen und/oder ein- bis zweifach durch Hydroxy, $(C_1\text{-}C_6)$Alkoxy, $(C_1\text{-}C_4)$Akoxy$(C_1\text{-}C_4)$alkoxy, $(C_1\text{-}C_4)$-Alkylthio, $(C_1\text{-}C_4)$-Alkylsulfinyl, $(C_1\text{-}C_4)$Alkylsulfonyl, Mono- ode Di-$(C_1\text{-}C_4\text{-alkyl})$amino, Cyano, Aminocarbonyl, $(C_1\text{-}C_4)$-Alkylcarbonyl, $(C_1\text{-}C_4\text{-Alkoxy})$carbonyl, Cyclo$(C_3\text{-}C_7)$-alkyl, Tri$(C_1\text{-}C_4)$alkyl-silyl, Benzyloxy, Benzyloxyethoxy, Phenyl, Phenyl, das durch Halogen oder $(C_1\text{-}C_4)$Alkyl substituiert ist, durch Phenoxy, Phenylthio, die durch Halogen oder $(C_1\text{-}C_4)$-Alkyl substituiert sein können, durch Oxiranyl, Tetrahydrofuryl, Triazolyl, Pyridinyl, Imidazolyl, durch Carboxy, Carboxylat mit einem für die Landwirtschaft einsetzbaren Kation oder durch den Rest -O-N=$C(CH_3)_2$ substituiert ist, $(C_3\text{-}C_6)$Alkenyl, $(C_3\text{-}C_6)$-Halogenalkenyl, unsubstituiertes oder durch Halogen oder $(C_1\text{-}C_4)$Alkyl substituiertes Cyclo$(C_3\text{-}C_7)$-alkyl, unsubstituiertes oder durch Halogen oder $(C_1\text{-}C_4)$Alkyl substituiertes Cyclo$(C_5\text{-}C_7)$alkenyl, $(C_3\text{-}C_6)$Alkinyl, 1,2-Epoxy-prop-3-yl, Phenyl oder Phenyl, das ein oder zweifach durch Halogen, Nitro, Cyano, $(C_1\text{-}C_4)$Alkyl, $(C_1\text{-}C_4\text{-Alkoxy})$carbonyl oder $(C_1\text{-}C_4)$Alkoxy substituiert ist, $(C_1\text{-}C_4\text{-Alkyl})$-carbonyl, Phenylcarbonyl, wobei der Phenylring durch Halogen, Nitro, Cyano oder $(C_1\text{-}C_4)$Alkyl substituiert sein kann, einen Rest der Formeln

$-N=C(R^{10})_2$ , $-NR^3R^{11}$, 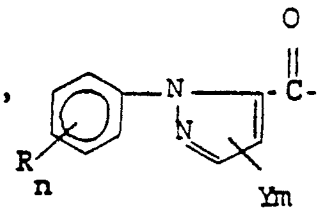 , 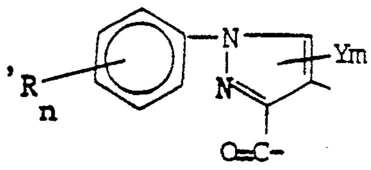

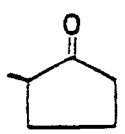 , 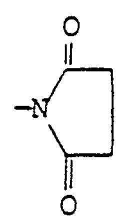 , 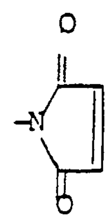 , 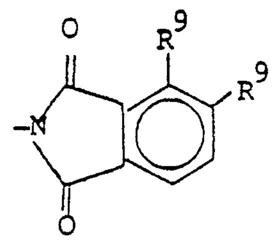

oder ein für die Landwirtschaft einsetzbares Kation,

$R^2$ ($C_1$-$C_{12}$)Alkyl oder ($C_1$-$C_{12}$)Alkyl, das bis zu zweifach durch ($C_1$-$C_4$)Alkoxyethoxy , Cyclo($C_3$-$C_6$)-alkyl, Benzyloxy, Phenyl, Phenoxy, ($C_1$-$C_4$)Alkylthio, ($C_1$-$C_4$-Alkoxy)-carbonyl, Carboxy oder Carboxylat mit einem für die Landwirtschaft einsetzbares Kation, substituiert ist,

$R^3$ jeweils unabhängig voneinander ($C_1$-$C_6$)-Alkyl, Phenyl oder ($C_3$-$C_6$)-Alkenyl,

$R^4$ Wasserstoff, ($C_1$-$C_{12}$)Alkyl oder ($C_1$-$C_{12}$)Alkyl, das bis zu zweifach durch ($C_1$-$C_6$)Alkoxy, ($C_1$-$C_4$)-Alkoxy-ethoxy, Hydroxy, Hydroxyimino, ($C_1$-$C_4$)-Alkoxyimino, Halogen, Cyclo($C_3$-$C_6$)alkyl, Benzyloxy, Cyano, Aminocarbonyl, Carboxy, ($C_1$-$C_4$-Alkoxy)-carbonyl, Formyl, Phenyl oder Phenoxy substituiert ist, Phenyl oder Phenyl, das bis zu zweifach durch Halogen, Nitro, Cyano, ($C_1$-$C_4$)Alkyl oder ($C_1$-$C_4$)-Alkoxy substituiert ist; ($C_3$-$C_6$)-Alkenyl, ($C_3$-$C_6$)Cycloalkyl, einen Rest der Formeln $-NR^3R^{12}$, $-O-R^6$, $-NH-CONH_2$, $-NH-CS-NH_2$ oder $-SO_2R^{13}$ oder

$R^3$ und $R^4$ gemeinsam mit dem Stickstoffatom an das sie gebunden sind, einen gesättigten oder ungesättigten gegebenenfalls benzokonensierten drei- bis siebengliedrigen Ring, der bis zu drei Heteroatome aus der Gruppe O, N oder S enthält und der unsubstituiert oder durch ($C_1$-$C_4$)Alkyl oder Halogen substituiert ist und eine Carbonylgruppe enthalten kann,

$R^5$ H, ($C_1$-$C_6$)Alkyl oder Phenyl, oder im Falle R = $-CS-OR^5$ ein für die Landwirtschaft einsetzbares Kation,

$R^6$ jeweils unabhängig voneinander H, ($C_1$-$C_4$)Alkyl oder Benzyl,

$R^7$ jeweils unabhängig voneinander H, ($C_1$-$C_{12}$)Alkyl, das unsubstituiert oder durch Phenyl, das unsubstituiert oder durch Halogen, Nitro, Cyano, ($C_1$-$C_4$)Alkyl oder ($C_1$-$C_4$)Alkoxy substituiert ist, durch Hydroxy, Cyano, ($C_1$-$C_4$-Alkoxy)-carbonyl, ($C_1$-$C_4$)-Alkylthio, ($C_1$-$C_4$)-Alkoxy, Cyclo($C_5$-$C_7$)alkyl oder Benzyloxy substituiert ist, ($C_3$-$C_6$)Alkenyl, Halogen($C_3$-$C_6$)Alkenyl, Halogen ($C_3$-$C_6$)-alkenyl, ($C_3$-$C_6$)Alkinyl, Cyclo($C_5$-$C_8$)alkyl, Cyclo($C_5$-$C_6$)alkenyl, ($C_1$-$C_6$-Alkyl)carbonyl, Halogen($C_1$-$C_6$-alkyl)-carbonyl, [($C_1$-$C_6$-Alkyl)amino]carbonyl, Benzoyl, Halogenbenzoyl oder Methylbenzoyl

$R^8$ jeweils unabhängig voneinander ($C_1$-$C_6$)Alkyl, das unsubstituiertes oder durch Phenyl, Cyclo($C_5$-$C_7$)alkyl, ($C_1$-$C_4$)Alkoxy, ($C_1$-$C_4$)Alkylthio oder Halogen substituiert ist, oder zwei Reste $R^8$ gemeinsam mit Z und dem Kohlenstoffatom, an das sie gebunden sind, einen unsubstituierten oder durch ($C_1$-$C_4$)Alkyl, Hydroxy-($C_1$-$C_4$)alkyl, Halogen($C_1$-$C_4$)alkyl oder Phenyl substituierten 5- oder 6-gliedrigen gesättigten heterocyclischen Ring;

$R^9$ jeweils unabhängig voneinander H, Halogen, ($C_1$-$C_4$)-Alkyl, Nitro oder Cyano,

$R^{10}$ unabhängig voneinander H, ($C_1$-$C_6$)Alkyl, das unsubstituiert oder durch ($C_1$-$C_4$)Alkoxy, Triazolyl oder Imidazolyl substituiert ist, Cyclo($C_3$-$C_6$)alkyl, ($C_3$-$C_6$)Alkenyl, Phenyl oder Benzyl, oder beide Reste $R^{10}$ gemeinsam mit dem Kohlenstoffatom, an das sie gebunden sind, ein unsubstituiertes oder durch Methyl oder Halogen substituiertes Cyclo-($C_5$-$C_7$)alkyl,

$R^{11}$ ($C_1$-$C_4$)Alkyl, Phenyl, ($C_1$-$C_6$-Alkyl)carbonyl, Benzyl, Benzoyl, Halogenbenzyl, Halogenbenzoyl oder Methylbenzoyl,

$R^{12}$ H, ($C_1$-$C_4$)Alkyl, Formyl, ($C_1$-$C_6$-Alkyl)carbonyl, Benzoyl, Halogenbenzoyl, Methylbenzoyl oder Trihalogenacetyl,

$R^{13}$ ($C_1$-$C_4$)Alkyl, Phenyl oder Methylphenyl,

m 0 oder 1,

n eine ganze Zahl von 0 bis 5,

p eine ganze Zahl von 0 bis 4, und
q eine ganze Zahl von 0 bis 6 bedeute,
dadurch gekennzeichnet, daß man eine Verbindung der Formel II

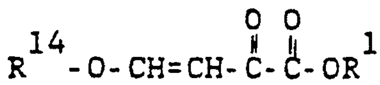 (II),

worin $R^{14}$ $(C_1\text{-}C_6)$Alkyl bedeutet, mit einer Verbindung der Formel III

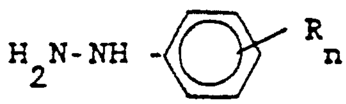 (III)

umsetzt und anschließend gegebenenfalls derivatisiert.

2. Pflanzenbehandlungsmittel, gekennzeichnet durch einen wirksamen Gehalt einer Verbindung der Formel I von Anspruch 1.

3. Pflanzenwachstumsregulierende Mittel, gekennzeichnet durch einen wirksamen Gehalt einer Verbindung der Formel I von Anspruch 1.

4. Mittel zum Schutz von Kulturpflanzen vor phytotoxischen Nebenwirkungen von Herbiziden, gekennzeichnet durch einen wirksamen Gehalt einer Verbindung der Formel I von Anspruch 1.

5. Verwendung der Verbindungen der Formel I zur Wachstumsregulierung von Pflanzen.

6. Verwendung der Verbindungen der Formel I zum Schutz von Kulturpflanzen vor phytotoxischen Nebenwirkungen von Herbiziden.

7. Verfahren zur Regulierung des Pflanzenwachstums, dadurch gekennzeichnet, daß man auf die Pflanzen oder die Anbaufläche eine wirksame Menge einer Verbindung der Formel I appliziert.

8. Verfahren zum Schutz von Kulturpflanzen vor phytotoxischen Nebenwirkungen von Herbiziden, dadurch gekennzeichnet, daß man auf die Kulturpflanzen oder die Anbaufläche eine wirksame Menge einer Verbindung der Formel I vor, nach oder gleichzeitig mit dem Herbizid appliziert.

9. Verfahren gemäß Anspruch 8, dadurch gekennzeichnet, daß das Herbizid ein Phenoxy-phenoxy- oder Heteroaryloxyphenoxy-carbonsäureester ist.

## Claims

1. A compound of the formula I, and the salts and quaternization products thereof,

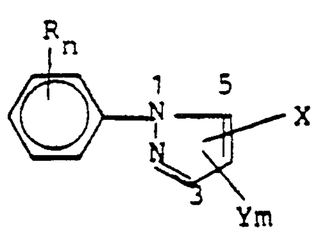 (I),

in which
R, in each case independently of one another, denote halogen, hydroxyl, cyano, nitro, $(C_1\text{-}C_4)$alkyl,

halo($C_1$-$C_4$)alkyl, ($C_1$-$C_4$)alkoxy ($C_1$-$C_4$)alkyl, ($C_1$-$C_6$) alkoxy, ($C_1$-$C_6$) alkoxy $C_1$-$C_4$) alkoxy, halo($C_1$-$C_6$)-alkoxy, ($C_1$-$C_4$)alkylthio, halo($C_1$-$C_4$)alkylthio, carboxyl, ($C_1$-$C_4$)alkoxycarbonyl, ($C_1$-$C_4$)-alkylsulfinyl, halo($C_1$-$C_4$)alkylsulfinyl, ($C_1$-$C_4$)alkylsulfonyl, halo($C_1$-$C_4$)alkylsulfonyl, ($C_1$-$C_4$)-alkylsulfonyloxy, halo($C_1$-$C_4$)alkylsulfonyloxy, phenyl, halophenyl, phenoxy or halophenoxy,
X is oriented in the 3- or 5-position of the pyrazole ring and denotes a radical of the formulae

-CN, 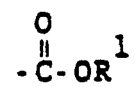, 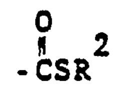, 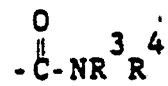,

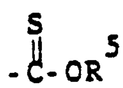, 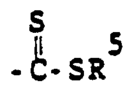, 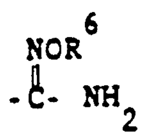, 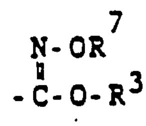

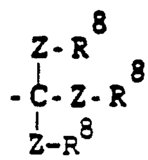, 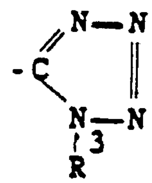, 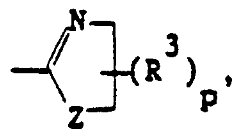, 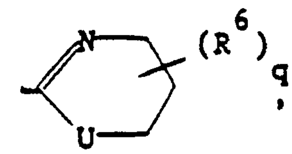,

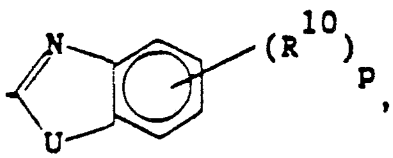, 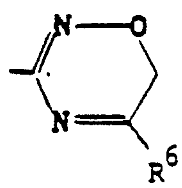 oder 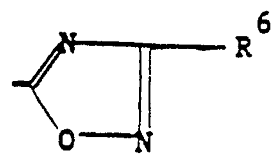

Y denotes halogen,
Z denotes O or S,
U denotes O, S or N-$R^6$,
$R^1$ denotes hydrogen, ($C_1$-$C_{12}$)alkyl, ($C_1$-$C_{12}$)alkyl which is monosubstituted or polysubstituted by halogen and/or monosubstituted or disubstituted by hydroxyl, (C,-$C_6$) alkoxy, ($C_1$-$C_4$) alkoxy-($C_1$-$C_4$) alkoxy, ($C_1$-$C_4$)alkylthio, ($C_1$-$C_4$)alkylsulfinyl, ($C_1$-$C_4$)alkyl-sulfonyl, mono- or di(C,-$C_4$-alkyl)amino, cyano, aminocarbonyl, ($C_1$-$C_4$)alkylcarbonyl, ($C_1$-$C_4$-alkoxy)-carbonyl, cyclo($C_3$-$C_7$)alkyl, tri($C_1$-$C_4$)-alkylsilyl, benzyloxy, benzyloxyethoxy, phenyl, phenyl which is substituted by halogen or ($C_1$-$C_4$)-alkyl, phenoxy or phenylthio which may be substituted by halogen or ($C_1$-$C_4$)alkyl, oxiranyl, tetrahydrofuryl, triazolyl, pyridinyl, imidazolyl, carboxyl, carboxylate with a cation which can be employed for agriculture, or the -O-N = $C(CH_3)_2$ radical, denotes ($C_3$-$C_6$)alkenyl, halo($C_3$-$C_6$)alkenyl, cyclo($C_3$-$C_7$)alkyl which is unsubstituted or substituted by halogen or ($C_1$-$C_4$)alkyl, cyclo($C_5$-$C_7$)-alkenyl which is unsubstituted or substituted by halogen or ($C_1$-$C_4$)alkyl, ($C_3$-$C_6$)alkynyl, 1,2-epoxyprop-3-yl, phenyl or phenyl which is monosubstituted or disubstituted by halogen, nitro, cyano, ($C_1$-$C_4$)alkyl, ($C_1$-$C_4$-alkoxy)carbonyl or ($C_1$-$C_4$)alkoxy, ($C_1$-$C_4$-alkyl)carbonyl, phenylcarbonyl where the phenyl ring may be substituted by halogen, nitro, cyano or ($C_1$-$C_4$)alkyl, a radical of the formulae

$-N=C(R^{10})_2$ , $-NR^3R^{11}$, 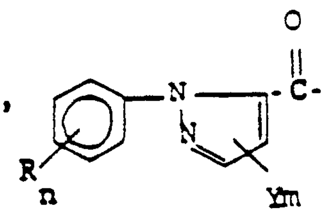 , 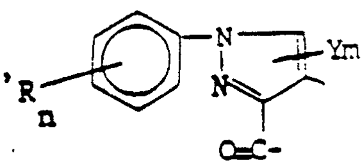

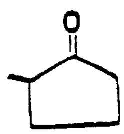 , 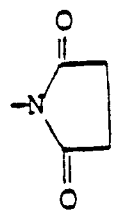 , 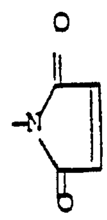 , 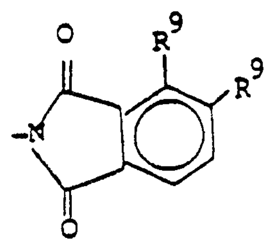

or a cation which can be employed for agriculture,

$R^2$ denotes $(C_1-C_{12})$alkyl or $(C_1-C_{12})$alkyl which is monosubstituted or disubstituted by$(C_1-C_4)$-alkoxyethoxy, cyclo$(C_3-C_6)$alkyl, benzyloxy, phenyl, phenoxy, $(C_1-C_4)$-alkylthio, $(C_1-C_4$-alkoxy)-carbonyl, carboxyl or carboxylate with a cation which can be employed for agriculture,

$R^3$, in each case independently of one another, denote $(C_1-C_6)$alkyl, phenyl or $(C_3-C_6)$alkenyl,

$R^4$ denotes hydrogen, $(C_1-C_{12})$alkyl or $(C_1-C_{12})$alkyl which is monosubstituted or disubstituted by $(C_1-C_6)$alkoxy, $(C_1-C_4)$alkoxyethoxy, hydroxyl, hydroxyimino, $(C_1-C_4)$alkoxyimino, halogen, cyclo$(C_3-C_6)$-alkyl, benzyloxy, cyano, aminocarbonyl, carboxyl, $(C_1-C_4$-alkoxy)carbonyl, formyl, phenyl or phenoxy, denotes phenyl or phenyl which is monosubstituted or disubstituted by halogen, nitro, cyano, $(C_1-C_4)$alkyl or $(C_1-C_4)$alkoxy; $(C_3-C_6)$alkenyl, $(C_3-C_6)$cycloalkyl, or a radical of the formulae $-NR^3R^{12}$,$-O-R^6$,$-NH-CONH_2$,$-NH-CS-NH_2$ or $-SO_2R^{13}$ or

$R^3$ and $R^4$, together with the nitrogen atom to which they are bound, denote a saturated or unsaturated, optionally benzo-fused three- to seven-membered ring which contains up to three heteroatoms from the group comprising O, N or S and which is unsubstituted or substituted by $(C_1-C_4)$alkyl or halogen and may contain a carbonyl group,

$R^5$ denotes H, $(C_1-C_6)$alkyl or phenyl, or in the case where x = $-CS-OR^5$, denotes a cation which can be employed for agriculture,

$R^6$, in each case independently of one another, denote H, $(C_1-C_4)$alkyl or benzyl,

$R^7$, in each case independently of one another, denote H, $(C_1-C_{12})$alkyl which is unsubstituted or substituted by phenyl which is unsubstituted or substituted by halogen, nitro, cyano, $(C_1-C_4)$alkyl or $(C_1-C_4)$alkoxy, by hydroxyl, cyano, $(C_1-C_4$-alkoxy)carbonyl, $(C_1-C_4)$alkylthio, $(C_1-C_4)$alkoxy, cyclo$(C_5-C_7)$alkyl or benzyloxy, denotes $(C_3-C_6)$alkenyl, halo$(C_3-C_6)$alkenyl, $(C_3-C_6)$alkynyl, cyclo$(C_5-C_8)$alkyl, cyclo$(C_5-C_6)$alkenyl, $(C_1-C_6$-alkyl)carbonyl, halo$(C_1-C_6$-alkyl)carbonyl, [$(C_1-C_6$-alkyl)amino]carbonyl, benzoyl, halobenzoyl or methylbenzoyl,

$R^8$, in each case independently of one another, denote $(C_1-C_6)$alkyl which is unsubstituted or substituted by phenyl, cyclo$(C_5-C_7)$alkyl, $(C_1-C_4)$alkoxy, $(C_1-C_4)$alkylthio or halogen, or two radicals $R^8$, together with Z and the carbon atom to which they are bound, denote a 5- or 6-membered saturated heterocyclic ring which is unsubstituted or substituted by $(C_1-C_4)$alkyl, hydroxy$(C_1-C_4)$alkyl, halo$(C_1-C_4)$alkyl or phenyl;

$R^9$, in each case independently of one another, denote H, halogen, $(C_1-C_4)$alkyl, nitro or cyano,

$R^{10}$, in each case independently of one another, denote H, $(C_1-C_6)$alkyl which is unsubstituted or substituted by $(C_1-C_4)$alkoxy, triazolyl or imidazolyl, cyclo$(C_3-C_6)$alkyl, $(C_3-C_6)$alkenyl, phenyl or benzyl, or, where $R^1$ = $-N=C(R^{10})_2$, both radicals $R^{10}$, together with the carbon atom to which they are bound, denote a cyclo$(C_5-C_7)$alkyl which is unsubstituted or substituted by methyl or halogen,

$R^{11}$ denotes $(C_1-C_4)$alkyl, phenyl, $(C_1-C_6$-alkyl) carbonyl, benzyl, benzoyl, halobenzyl, halobenzoyl or methylbenzoyl,

$R^{12}$ denotes H, $(C_1-C_4)$alkyl, formyl, $(C_1-C_6$-alkyl)carbonyl, benzoyl, halobenzoyl, methylbenzoyl or trihaloacetyl,

$R^{13}$ denotes $(C_1-C_4)$alkyl, phenyl or methylphenyl,

m denotes 0 or 1,

n denotes an integer from 0 to 5,

p denotes an integer from 0 to 4 and
q denotes an integer from 0 to 6.

2. A process for the preparation of the compounds of the formula I as claimed in claim 1, which comprises reacting a compound of the formula II

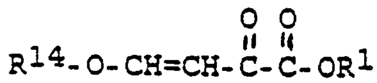

(II),

in which $R^{14}$ denotes ($C_1$-$C_6$)alkyl, with a compound of the formula (III)

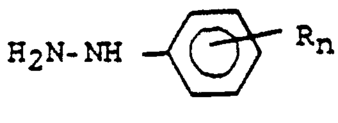

(III)

and subsequently derivatizing it, if appropriate.

3. A plant-treatment agent, containing an active content of a compound of the formula I as claimed in claim 1.

4. A plant growth regulator, containing an active content cf a compound of the formula I as claimed in claim 1.

5. An agent for protecting crop plants against phytotoxic side effects of herbicides, containing an active content of a compound of the formula I as claimed in claim 1.

6. The use of a compound of the formula I as claimed in claim 1 for regulating the growth of plants.

7. The use of a compound of the formula I as claimed in claim 1 for protecting crop plants against phytotoxic side effects of herbicides.

8. A process for regulating plant growth, wherein an effective amount of a compound of the formula I as claimed in claim 1 is applied to the plants or to the cultivated area.

9. A process for protecting crop plants against phytotoxic side effects of herbicides, wherein an effective amount of a compound of the formula I as claimed in claim 1 is applied to the crop plants or the cultivated area before, after or at the same time as the herbicide.

10. The process as claimed in claim 9, wherein the herbicide is a phenoxyphenoxy- or heteroaryloxyphenoxy-containing herbicide.

Claims for the following Contracting States : AT, ES

1. A process for the preparation of a compound of the formula I,

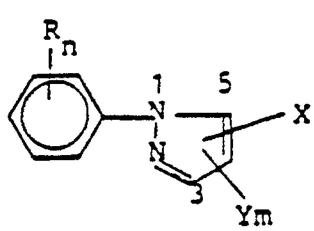

(I),

in which

R, in each case independently of one another, denote halogen, hydroxyl, cyano, nitro, ($C_1$-$C_4$)alkyl,

halo($C_1$-$C_4$)alkyl, ($C_1$-$C_4$)alkoxy($C_1$-$C_4$)alkyl, ($C_1$-$C_6$) alkoxy, ($C_1$-$C_6$)alkoxy($C_1$-$C_4$)alkoxy, halo($C_1$-$C_6$)-alkoxy, ($C_1$-$C_4$)alkylthio, halo($C_1$-$C_4$)alkylthio, carboxyl, ($C_1$-$C_4$)alkoxycarbonyl, ($C_1$-$C_4$)alkylsulfinyl, halo($C_1$-$C_4$)alkylsulfinyl, ($C_1$-$C_4$)alkylsulfonyl, halo($C_1$-$C_4$)alkylsulfonyl, ($C_1$-$C_4$)alkylsulfonyloxy, halo ($C_1$-$C_4$)alkylsulfonyloxy, phenyl, halophenyl, phenoxy or halophenoxy,

X is oriented in the 3- or 5-position of the pyrazole ring and denotes a radical of the formulae

-CN, 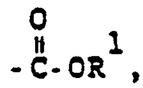, 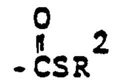, 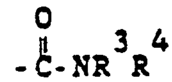,

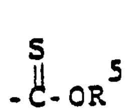, 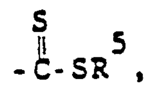, 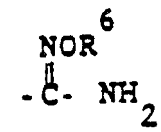, 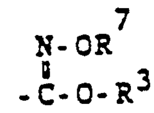

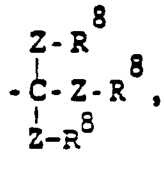, 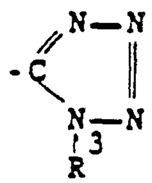, 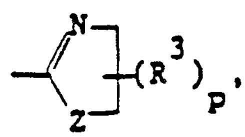, 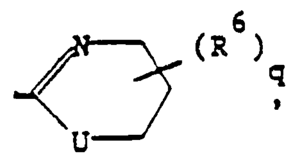,

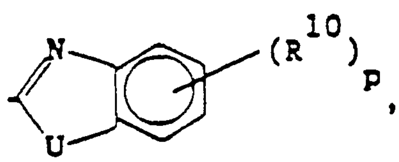, 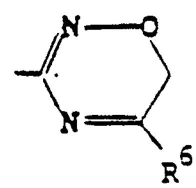 oder 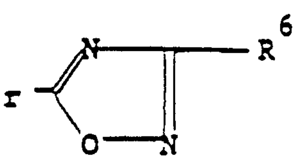

Y denotes halogen,
Z denotes O or S,
U denotes O, S or N-$R^6$,
$R^1$ denotes hydrogen, ($C_1$-$C_{12}$)alkyl, ($C_1$-$C_{12}$)alkyl which is monosubstituted or polysubstituted by halogen and/or monosubstituted or disubstituted by hydroxyl, ($C_1$-$C_6$)alkoxy, ($C_1$-$C_4$)alkoxy($C_1$-$C_4$)-alkoxy, ($C_1$-$C_4$)alkylthio, ($C_1$-$C_4$)alkylsulfinyl, ($C_1$-$C_4$)alkylsulfonyl, mono- or di-($C_1$-$C_4$-alkyl)amino, cyano, aminocarbonyl, ($C_1$-$C_4$)alkylcarbonyl, ($C_1$-$C_4$-alkoxy)carbonyl, cyclo($C_3$-$C_7$)alkyl, tri($C_1$-$C_4$)-alkylsilyl, benzyloxy, benzyloxyethoxy, phenyl, phenyl which is substituted by halogen or ($C_1$-$C_4$)-alkyl, phenoxy or phenylthio which may be substituted by halogen or ($C_1$-$C_4$)alkyl, oxiranyl, tetrahydrofuryl, triazolyl, pyridinyl, imidazolyl, carboxyl, carboxylate with a cation which can be employed for agriculture, or the -0-N=C($CH_3$)$_2$, radical, denotes ($C_3$-$C_6$)alkenyl, halo($C_3$-$C_6$)alkenyl, cyclo($C_3$-$C_7$)alkyl which is unsubstituted or substituted by halogen or ($C_1$-$C_4$)alkyl, cyclo($C_5$-$C_7$)-alkenyl which is unsubstituted or substituted by halogen or ($C_1$-$C_4$)alkyl, ($C_3$-$C_6$)alkynyl, 1,2-epoxyprop-3-yl, phenyl or phenyl which is monosubstituted or disubstituted by halogen, nitro, cyano, ($C_1$-$C_4$)alkyl, ($C_1$-$C_4$-alkoxy)carbonyl or ($C_1$-$C_4$)-alkoxy, ($C_1$-$C_4$-alkyl)carbonyl, phenylcarbonyl where the phenyl ring may be substituted by halogen, nitro, cyano or ($C_1$-$C_4$)alkyl, a radical of the formulae

$-N=C(R^{10})_2$ , $-NR^3R^{11}$, 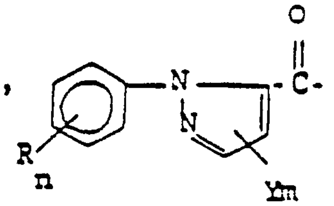 , 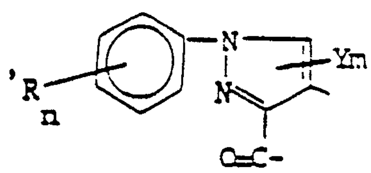

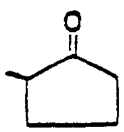 , 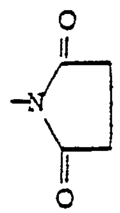 , 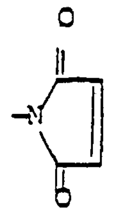 , 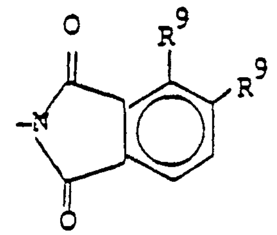 .

or a cation which can be employed for agriculture,

$R^2$ denotes $(C_1-C_{12})$alkyl or $(C_1-C_{12})$alkyl which is monosubstituted or disubstituted by $(C_1-C_4)$-alkoxyethoxy, cyclo$(C_3-C_6)$alkyl, benzyloxy, phenyl, phenoxy, $(C_1-C_4)$alkylthio, $(C_1-C_4$-alkoxy)-carbonyl, carboxyl or carboxylate with a cation which can be employed for agriculture,

$R^3$, in each case independently of one another, denote $(C_1-C_6)$alkyl, phenyl or $(C_3-C_6)$alkenyl,

$R^4$ denotes hydrogen, $(C_1-C_{12})$alkyl or $(C_1-C_{12})$alkyl which is monosubstituted or disubstituted by $(C_1-C_6)$alkoxy, $(C_1-C_4)$alkoxyethoxy, hydroxyl, hydroxyimino, $(C_1-C_4)$alkoxyimino, halogen, cyclo$(C_3-C_6)$alkyl, benzyloxy, cyano, aminocarbonyl, carboxyl, $(C_1-C_4$-alkoxy)carbonyl, formyl, phenyl or phenoxy, denotes phenyl or phenyl which is monosubstituted or disubstituted by halogen, nitro, cyano, $(C_1-C_4)$alkyl or $(C_1-C_4)$alkoxy; $(C_3-C_6)$alkenyl, $(C_3-C_6)$cycloalkyl, or a radical of the formulae $-NR^3R^{12}$,$-O-R^6$,$-NH-CONH_2$,$-NH-CS-NH_2$ or $-SO_2R^{13}$ or

$R^3$ and $R^4$, together with the nitrogen atom to which they are bound, denote a saturated or unsaturated, optionally benzo-fused three- to seven-membered ring which contains up to three heteroatoms from the group comprising O, N or S and which is unsubstituted or substituted by $(C_1-C_4)$alkyl or halogen and may contain a carbonyl group,

$R^5$ denotes H, $(C_1-C_6)$alkyl or phenyl, or in the case where R = $-CS-OR^5$, denotes a cation which can be employed for agriculture,

$R^6$ in each case independently of one another, denote H, $(C_1-C_4)$alkyl or benzyl,

$R^7$, in each case independently of one another, denote H, $(C_1-C_{12})$alkyl which is unsubstituted or substituted by phenyl which is unsubstituted or substituted by halogen, nitro, cyano, $(C_1-C_4)$alkyl or $(C_1-C_4)$alkoxy, by hydroxyl, cyano, $(C_1-C_4$-alkoxy)carbonyl, $(C_1-C_4)$alkylthio, $(C_1-C_4)$alkoxy, cyclo$(C_5-C_7)$alkyl or benzyloxy, denotes $(C_3-C_6)$-alkenyl, halo$(C_3-C_6)$alkenyl, $(C_3-C_6)$alkynyl, cyclo$(C_5-C_8)$alkyl, cyclo$(C_5-C_6)$alkenyl, $(C_1-C_6)$-alkyl)carbonyl, halo$(C_1-C_6$-alkyl)carbonyl, $[(C_1-C_6$-alkyl)amino]carbonyl, benzoyl, halobenzoyl or methylbenzoyl,

$R^8$, in each case independently of one another, denote $(C_1-C_6)$alkyl which is unsubstituted or substituted by phenyl, cyclo$(C_5-C_7)$alkyl, $(C_1-C_4)$alkoxy, $(C_1-C_4)$alkylthio or halogen, or two radicals $R^8$, together with Z and the carbon atom to which they are bound, denote a 5- or 6-membered saturated heterocyclic ring which is unsubstituted or substituted by $(C_1-C_4)$alkyl, hydroxy$(C_1-C_4)$-alkyl, halo$(C_1-C_4)$alkyl or phenyl;

$R^9$, in each case independently of one another, denote H, halogen, $(C_1-C_4)$alkyl, nitro or cyano,

$R^{10}$, in each case independently of one another, denote H, $(C_1-C_6)$alkyl which is unsubstituted or substituted by $(C_1-C_4)$alkoxy, triazolyl or imidazolyl, cyclo$(C_3-C_6)$alkyl, $(C_3-C_6)$alkenyl, phenyl or benzyl, or both radicals $R^{10}$, together with the carbon atom to which they are bound, denote a cyclo-$(C_5-C_7)$alkyl which is unsubstituted or substituted by methyl or halogen,

$R^{11}$ denotes $(C_1-C_4)$alkyl, phenyl, $(C_1-C_6$-alkyl)carbonyl, benzyl, benzoyl, halobenzyl, halobenzoyl or methylbenzoyl,

$R^{12}$ denotes H, $(C_1-C_4)$alkyl, formyl, $(C_1-C_6$-alkyl)carbonyl, benzoyl, halobenzoyl, methylbenzoyl or trihaloacetyl,

$R^{13}$ denotes $(C_1-C_4)$alkyl, phenyl or methylphenyl,

m denotes 0 or 1,

n denotes an integer from 0 to 5,

p denotes an integer from 0 to 4 and
q denotes an integer from 0 to 6, which comprises reacting a compound of the formula II

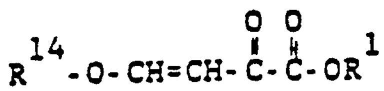

(II),

in which $R^{14}$ denotes $(C_1\text{-}C_6)$alkyl, with a compound of the formula (III)

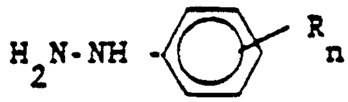

(III)

and subsequently derivatizing it, if appropriate.

2. A plant-treatment agent, containing an active content of a compound of the formula I as claimed in claim 1.

3. A plant growth regulator, containing an active content of a compound of the formula I as claimed in claim 1.

4. An agent for protecting crop plants against phytotoxic side effects of herbicides, containing an active content of a compound of the formula I as claimed in claim 1.

5. The use of a compound of the formula I as claimed in claim 1 for regulating the growth of plants.

6. The use of a compound of the formula I as claimed in claim 1 for protecting crop plants against phytotoxic side effects of herbicides.

7. A process for regulating plant growth, wherein an effective amount of a compound of the formula I as claimed in claim 1 is applied to the plants or to the cultivated area.

8. A process for protecting crop plants against phytotoxic side effects of herbicides, wherein an effective amount of a compound of the formula I as claimed in claim 1 is applied to the crop plants or the cultivated area before, after or at the same time as the herbicide.

9. The process as claimed in claim 8, wherein the herbicide is a phenoxyphenoxy- or heteroaryloxyphenoxy-carboxylate.

## Revendications

1. Composés de formule I, leurs sels et leurs produits de quaternisation :

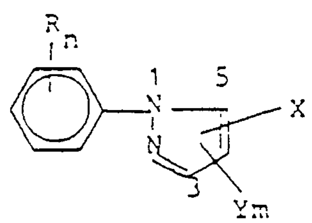

(I),

formule dans laquelle :
les R représentent chacun, indépendamment les uns des autres, un halogéne, un hydroxy, un cyano, un nitro, un alkyle en $C_1\text{-}C_4$, un halogénoalkyle en $C_1\text{-}C_4$, un $(C_1\text{-}C_4)$alcoxy-$(C_1\text{-}C_4)$alkyle, un

alcoxy en $C_1$-$C_6$, un ($C_1$-$C_6$)alcoxy-($C_1$-$C_4$)alcoxy, un halogénoalcoxy en $C_1$-$C_6$, un alkylthio en $C_1$-$C_4$, un halogeno-alkylthio en $C_1$-$C_4$, un carboxy, un ($C_1$-$C_4$)alcoxy-carbonyle, un alkylsulfinyle en $C_1$-$C_4$, un halogénoalkyl-sulfinyle en $C_1$-$C_4$, un alkylsulfonyle en $C_1$-$C_4$, un halogénoalkyl-sulfonyle en $C_1$-$C_4$, un alkylsulfonyloxy en $C_1$-$C_4$, un halogénoalkyl-sulfonyloxy en $C_1$-$C_4$, un phényle, un halogénophényle, un phénoxy ou un halogénophénoxy,

x occupe la position 3 ou la position 5 du cycle pyrazolique et représente un radical répondant a l'une des formules:

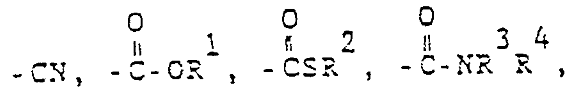

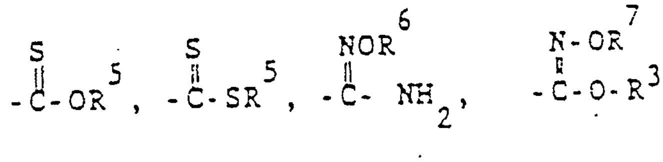

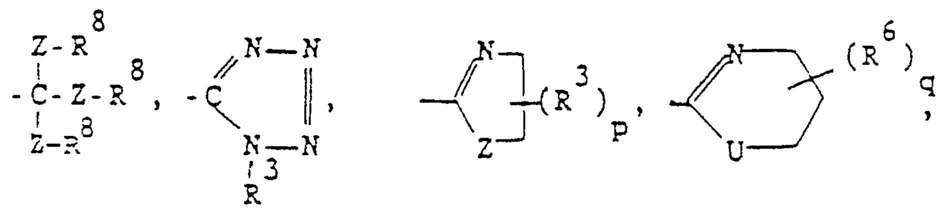

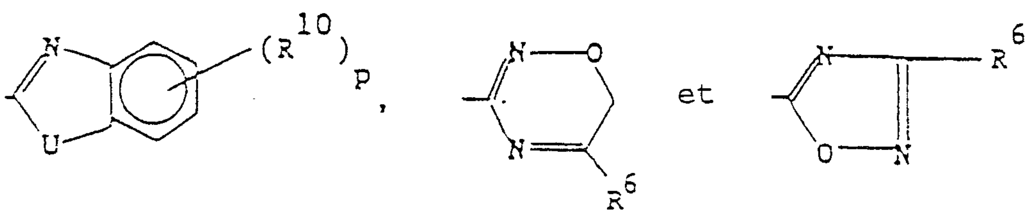

Y représente un halogène,

Z représente O ou S,

U représente O, S ou N-$R^6$,

$R^1$ représente :

- l'hydrogène,
- un alkyle en $C_1$-$C_{12}$,
- un alkyle en $C_1$-$C_{12}$ qui porte un ou plusieurs atomes d'halogènes et/ou un ou deux substituants pris dans l'ensemble constitué par l'hydroxy, les alcoxy en $C_1$-$C_6$, les ($C_1$-$C_4$)alcoxy-($C_1$-$C_4$)alcoxy, les alkylthio en $C_1$-$C_4$, les alkylsulfinyles en $C_1$-$C_4$, les alkylsulfonyles en $C_1$-$C_4$, les mono- et di-($C_1$-$C_4$)-alkyl-amino, le cyano, l'aminocarbonyle, les ($C_1$-$C_4$)alkylcarbonyles, les ($C_1$-$C_4$)-alcoxy-carbonyles, les cycloalkyles en $C_3$-$C_7$, les tri-($C_1$-$C_4$)alkyl-silyles, le benzyloxy, le benzyloxy-éthoxy, le phényle, les phényles porteurs d'un halogène ou d'un alkyle en $C_1$-$C_4$, le phénoxy, le phénylthio, ces deux derniers pouvant porter un halogène ou un alkyle en $C_1$-$C_4$, l'oxirannyle, les tétrahydrofuryles, les triazolyles, les pyridyles, les imidazolyles, le carboxy, les carboxylates formés avec des cations utilisables en agriculture et le radical -0-N=C($CH_3$)$_2$,
- un alcényle en $C_3$-$C_6$,
- un halogéno-alcényle en $C_3$-$C_6$,
- un cycloalkyle en $C_3$-$C_7$ non substitué ou porteur d'un halogène ou d'un alkyle en $C_1$-$C_4$,
- un cycloalcényle en $C_5$-$C_7$ non substitué ou porteur d'un halogène ou d'un alkyle en $C_1$-$C_4$,
- un alcynyle en $C_3$-$C_6$,
- un époxy-2,3 propyle,
- un phényle ou un phényle porteur d'un ou deux substituants pris dans l'ensemble constitué par les halogènes, le nitro, le cyano, les alkyles en $C_1$-$C_4$, les ($C_1$-$C_4$-alcoxy)-carbonyles et les alcoxy en $C_1$-$C_4$,
- un ($C_1$-$C_4$-alkyl)-carbonyle,
- un phénylcarbonyle dont le cycle phényle peut porter un halogène, un nitro, un cyano ou un

alkyle en $C_1$-$C_4$,

- un radical répondant a l'une des formules

$-N=C(R^{10})_2$ , $-NR^3R^{11}$, 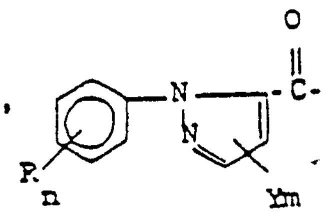 , 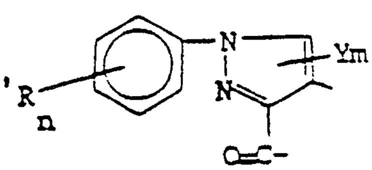

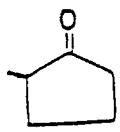 , 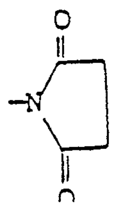 , 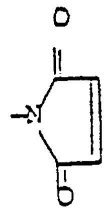 , 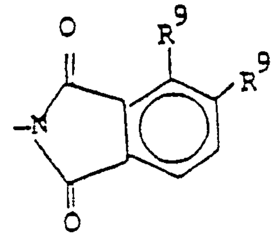

suivantes :

- un cation utilisable en agriculture,

$R^2$ représente un alkyle en $C_1$-$C_{12}$ ou un alkyle en $C_1$-$C_{12}$ qui porte au plus deux substituants pris dans l'ensemble constitué par les ($C_1$-$C_4$)alcoxy-éthoxy, les cycloalkyles en $C_3$-$C_6$, le benzyloxy, le phényle, le phénoxy, les alkylthio en $C_1$-$C_4$, les ($C_1$-$C_4$-alcoxy)-carbonyles, le carboxy et les carboxylates à cations utilisables en agriculture,

les $R^3$ représentent chacun, indépendamment les uns des autres, un alkyle en $C_1$-$C_6$, un phényle ou un alcényle en $C_3$-$C_6$,

$R^4$ représente :

- l'hydrogène,
- un alkyle en $C_1$-$C_{12}$,
- un alkyle en $C_1$-$C_{12}$ qui porte au plus deux substituants pris dans l'ensemble constitué par les alcoxy en $C_1$-$C_6$, les ($C_1$-$C_4$)alcoxy-éthoxy, l'hydroxy, l'hydroxy-imino, les alcoxy-imino en $C_1$-$C_4$, les halogènes, les cycloalkyles en $C_3$-$C_6$, le benzyloxy, le cyano, l'aminocarbonyle, le carboxy, les ($C_1$-$C_4$-alcoxy)-carbonyles, le formyle, le phényle et le phénoxy,
- un phényle,
- un phényle qui porte au plus deux substituants pris dans l'ensemble constitué par les halogènes, le nitro, le cyano, les alkyles en $C_1$-$C_4$ et les alcoxy en $C_1$-$C_4$,
- les alcényles en $c_3$-$c_6$,
- les cycloalkyles en $C_3$-$C_6$, et
- les radicaux répondant aux formules $-NR^3R^{12}$, $-O-R^6$, $-NH-CONH_2$, $-NH-CS-NH_2$ et $-SO_2R^{13}$, ou

$R^3$ et $R^4$ forment ensemble, et avec l'atome d'azote auquel ils sont liés, un cycle contenant de 3 à 7 maillons, saturé ou insaturé et éventuellement condensé a un noyau benzo, cycle qui contient au plus trois hétéroatomes pris dans l'ensemble constitué par O, N et S et qui ne porte pas de substituant ou porte un alkyle en $C_1$-$C_4$ ou un halogène, et qui peut contenir un radical carbonyle,

$R^5$ représente H, un alkyle en $C_1$-$C_6$ ou un phényle, ou encore, dans le cas ou X représente un radical $-CS-OR^5$, le symbole $R^5$ représente un cation utilisable en agriculture,

les $R^6$ représentent chacun, indépendamment les uns des autres, l'hydrogène, un alkyle en $C_1$-$C_4$ ou un benzyle,

les $R^7$ représentent chacun, indépendamment les uns des au-tres, l'hydrogène, un alkyle en $C_1$-$C_{12}$ non substitué ou porteur d'un phényle - lui-même éventuellement porteur d'un halogène, d'un nitro, d'un cyano, d'un alkyle en $C_1$-$C_4$ ou d'un alcoxy en ($C_1$-$C_4$) -, d'un hydroxy, d'un cyano, d'un ($C_1$-$C_4$-alcoxy)-carbonyle, d'un alkylthio en $C_1$-$C_4$, d'un alcoxy en $C_1$-$C_4$, d'un cycloalkyle en $C_5$-$C_7$ ou d'un benzyloxy, un alcényle en $C_3$-$C_6$, un halogéno-alcényle en $C_3$-$C_6$, un alcynyle en $C_3$-$C_6$, un cycloalkyle en $C_5$-$C_8$ un cyclo-alcényle en $C_5$ ou $C_6$, un ($C_1$-$C_6$-alkyl)-carbonyle, un halogéno-($C_1$-$C_6$-alkyl)-carbonyle, un [($C_1$-$C_6$-alkyl)-amino]-carbonyle, un benzoyle, un halogénobenzoyle ou un méthylbenzoyle,

les $R^8$ représentent chacun, indépendamment les uns des autres, un alkyle en $C_1$-$C_6$ qui ne porte pas de substituant ou qui porte un phényle, un cycloalkyle en $C_5$-$C_7$, un alcoxy en $C_1$-$C_4$, un

alkylthio en $C_1$-$C_4$ ou un halogène, ou deux radicaux $R^8$ forment ensemble, avec les Z et l'atome de carbone auxquels ils sont liés, un hétérocycle saturé a 5 ou 6 maillons, dépourvu de substituant ou porteur d'un alkyle en $C_1$-$C_4$, d'un hydroxyalkyle en $C_1$-$C_4$ d'un halogéno-alkyle en $C_1$-$C_4$ ou d'un phényle,
les $R^9$ représentent chacun, indépendamment les uns des autres, l'hydrogène, un halogène, un alkyle en $C_1$-$C_4$, un nitro ou un cyano,
les $R^{10}$ représentent chacun, indépendamment les uns des autres, l'hydrogène, un alkyle $C_1$-$C_6$ dépourvu de substituant ou porteur d'un alcoxy en $C_1$-$C_4$, d'un triazolyle ou d'un imidazolyle, un cycloalkyle en $C_3$-$C_6$, un alcényle en $C_3$-$C_6$, un phényle ou un benzyle, ou encore, dans le cas où $R^1$ est un radical $-N=C(R^{10})_2$, les deux radicaux $R^{10}$ forment ensemble et avec l'atome de carbone auquel ils sont liés un cycloalkyle en $C_5$-$C_7$ non substitué ou porteur d'un méthyle ou d'un halogène,
$R^{11}$ représente un alkyle en $C_1$-$C_4$, un phényle, un ($C_1$-$C_6$-alkyl)-carbonyle, un benzyle, un benzoyle, un halogénobenzyle, un halogénobenzoyle ou un méthylbenzoyle,
$R^{12}$ représente l'hydrogène, un alkyle en $C_1$-$C_4$, un formyle, un ($C_1$-$C_6$-alkyl)-carbonyle, un benzoyle, un halogénobenzoyle, un méthylbenzoyle ou un trihalogénoacétyle,
$R^{13}$ représente un alkyle en $C_1$-$C_4$, un phényle ou un méthylphényle,
m est égal à 0 ou à 1,
n désigne un nombre entier de 0 à 5
p désigne un nombre entier de 0 à 4 et
q désigne un nombre entier de 0 à 6.

2. Procédé pour préparer les composés de formule I selon la revendication 1, procédé caractérisé en ce qu'on fait réagir un composé répondant à la formule II :

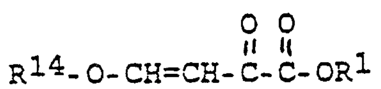

(II)

dans laquelle $R^{14}$ représente un alkyle en $C_1$-$C_6$, avec un composé de formule III :

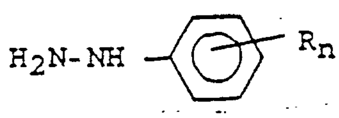

(III),

puis on transforme éventuellement le composé formé en l'un de ses dérivés.

3. Produits phytosanitaires caractérisés en ce qu'ils contiennent une quantité efficace d'un composé de formule I selon la revendication 1.

4. Produits régulateurs de croissance pour plantes, caractérisés en ce qu'ils contiennent une quantité efficace d'un composé de formule I selon la revendication 1.

5. Produits pour protéger des plantes de culture contre des actions secondaires phytotoxiques d'herbicides, produits caractérisés en ce qu'ils contiennent une quantité efficace d'un composé de formule I selon la revendication 1.

6. Application des composés de formule I selon la revendication 1 à la régulation de la croissance de plantes.

7. Application des composés de formule I selon la revendication 1 à la protection de plantes de culture contre des actions secondaires phytotoxiques d'herbicides.

8. Procédé pour régler la croissance de plantes, procédé caractérisé en ce qu'on applique sur les plantes ou sur la surface de culture une quantité efficace d'un composé de formule I selon la revendication 1.

9. Procédé pour protéger des plantes de culture contre des actions secondaires phytotoxiques d'herbicides, procédé caractérisé en ce qu'on applique sur les plantes de culture ou sur la surface de culture une quantité efficace d'un composé de formule I selon la revendication 1, avant, après ou en même

temps que l'herbicide.

**10.** Procédé selon la revendication 9, caractérisé en ce que l'herbicide est un ester d'acide phénoxyphénoxy-carboxylique ou d'acide hétéroaryloxy-phénoxy-carboxylique.

Revendications pour les Etats contractants suivants : AT, ES

**1.** Procédé pour préparer des composés répondant à la formule I :

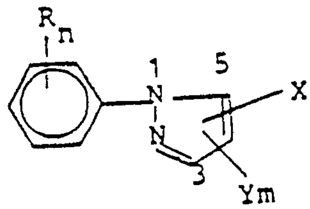 (I),

dans laquelle :

les R représentent chacun, indépendamment les uns des autres, un halogène, un hydroxy, un cyano, un nitro, un alkyle en $C_1$-$C_4$, un halogénoalkyle en $C_1$-$C_4$, un ($C_1$-$C_4$)alcoxy-($C_1$-$C_4$)alkyle, un alcoxy en $C_1$-$C_6$, un ($C_1$-$C_6$)alcoxy-($C_1$-$C_4$)alcoxy, un halogénoalcoxy en $C_1$-$C_6$, un alkylthio en $C_1$-$C_4$, un halogéno-alkylthio en $C_1$-$C_4$ , un carboxy, un ($C_1$-$C_4$)alcoxy-carbonyle, un alkylsulfinyle en $C_1$-$C_4$, un halogénoalkyl-sulfinyle en $C_1$-$C_4$, un alkylsulfonyle en $C_1$-$C_4$, un halogénoalkyl-sulfonyle en $C_1$-$C_4$, un alkylsulfonyloxy en $C_1$-$C_4$, un halogénoalkyl-sulfonyloxy en $C_1$-$C_4$, un phényle, un halogénophényle, un phénoxy ou un halogénophénoxy,

X occupe la position 3 ou la position 5 du cycle pyrazolique et représente un radical répondant à l'une des formules:

-CN, 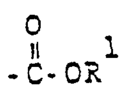 , 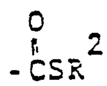 , 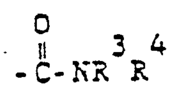 ,

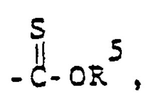 , 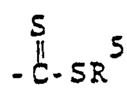 , 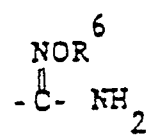 , 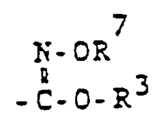

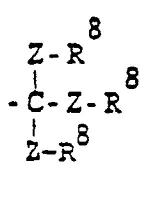 , 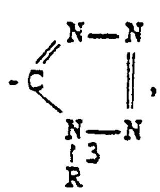 , 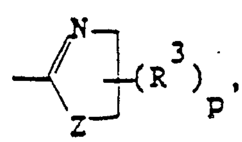 , 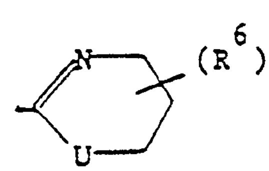 ,

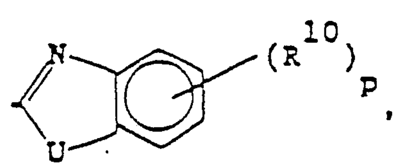 , 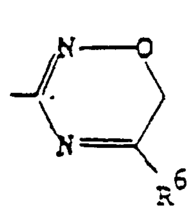 et 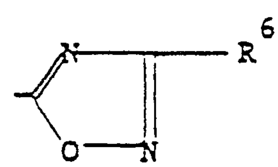

Y représente un halogène,
Z représente O ou S,
U représente O, S ou N-$R^6$,
$R^1$ représente :

- l'hydrogène,
- un alkyle en $C_1$-$C_{12}$,
- un alkyle en $C_1$-$C_{12}$ qui porte un ou plusieurs atomes d'halogènes et/ou un ou deux substituants pris dans l'ensemble constitué par l'hydroxy, les alcoxy en $C_1$-$C_6$, les ($C_1$-$C_4$)alcoxy-($C_1$-$C_4$)alcoxy, les alkylthio en $C_1$-$C_4$, les alkylsulfinyles en $C_1$-$C_4$, les alkylsulfonyles en $C_1$-$C_4$, les mono- et di-($C_1$-$C_4$)-alkyl-amino, le cyano, l'aminocarbonyle, les ($C_1$-$C_4$)alkylcarbonyles, les ($C_1$-$C_4$)-alcoxy-carbonyles, les cycloalkyles en $C_3$-$C_7$, les tri-($C_1$-$C_4$)alkyl-silyles, le benzyloxy, le benzyloxy-éthoxy, le phényle, les phényles porteurs d'un halogène ou d'un alkyle en $C_1$-$C_4$, le phénoxy, le phénylthio, ces deux derniers pouvant porter un halogène ou un alkyle en $C_1$-$C_4$, l'oxirannyle, les tétrahydrofuryles, les triazolyles, les pyridyles, les imidazolyles, le carboxy, les carboxylates formés avec des cations utilisables en agriculture et le radical $-O-N=C(CH_3)_2$,
- un alcényle en $C_3$-$C_6$,
- un halogéno-alcényle en $C_3$-$C_6$,
- un cycloalkyle en $C_3$-$C_7$ non substitué ou porteur d'un halogène ou d'un alkyle en $C_1$-$C_4$,
- un cycloalcényle en $C_5$-$C_7$ non substitué ou porteur d'un halogène ou d'un alkyle en $C_1$-$C_4$,
- un alcynyle en $C_3$-$C_6$,
- un époxy-2,3 propyle,
- un phényle ou un phényle porteur d'un ou deux substituants pris dans l'ensemble constitué par les halogènes, le nitro, le cyano, les alkyles en $C_1$-$C_4$, les ($C_1$-$C_4$-alcoxy)-carbonyles et les alcoxy en $C_1$-$C_4$,
- un ($C_1$-$C_4$-alkyl)-carbonyle,
- un phénylcarbonyle dont le cycle phényle peut porter un halogène, un nitro, un cyano ou un alkyle en $C_1$-$C_4$,
- un radical répondant à l'une des formules

$-N=C(R^{10})_2$ , $-NR^3R^{11}$, 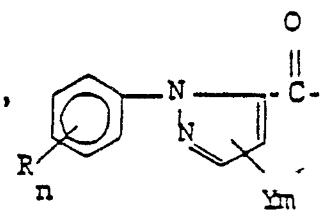 , 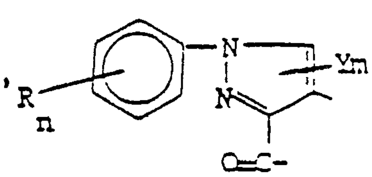

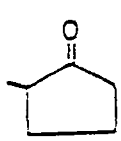 , 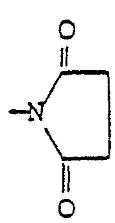 , 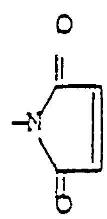 , 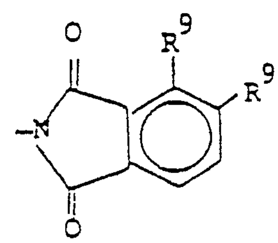

suivantes :
- un cation utilisable en agriculture,

$R^2$ représente un alkyle en $C_1$-$C_{12}$ ou un alkyle en $C_1$-$C_{12}$ qui porte au plus deux substituants pris dans l'ensemble constitué par les ($C_1$-$C_4$)alcoxy-éthoxy, les cycloalkyles en $C_3$-$C_6$, le benzyloxy, le phényle, le phénoxy, les alkylthio en $C_1$-$C_4$, les ($C_1$-$C_4$-alcoxy)-carbonyles, le carboxy et les carboxylates à cations utilisables en agriculture,

les $R^3$ représentent chacun, indépendamment les uns des autres, un alkyle en $C_1$-$C_6$, un phényle ou un alcényle en $C_3$-$C_6$,

$R^4$ représente :
- l'hydrogène,
- un alkyle en $C_1$-$C_{12}$,
- un alkyle en $C_1$-$C_{12}$ qui porte au Plus deux substituants pris dans l'ensemble constitué par les alcoxy en $C_1$-$C_6$, les ($C_1$-$C_4$)alcoxy-éthoxy, l'hydroxy, l'hydroxy-imino, les alcoxy-imino en $C_1$-$C_4$, les halogènes, les cycloalkyles en $C_3$-$C_6$, le benzyloxy, le cyano, l'aminocarbonyle, le carboxy, les ($C_1$-$C_4$-alcoxy)-carbonyles, le formyle, le phényle et le phénoxy,
- un phényle,

- un phényle qui porte au plus deux substituants pris dans l'ensemble constitué par les halogènes, le nitro, le cyano, les alkyles en $C_1$-$C_4$ et les alcoxy en $C_1$-$C_4$,
- les alcényles en $C_3$-$C_6$,
- les cycloalkyles en $C_3$-$C_6$ , et
- les radicaux répondant aux formules : $-NR^3R^{12}$, $-O-R^6$, $-NH-CONH_2$ , $-NH-CS-NH_2$ et $-SO_2R^{13}$ ,

ou
$R^3$ et $R^4$ forment ensemble, et avec l'atome d'azote auquel ils sont liés, un cycle contenant de 3 à 7 maillons, saturé ou insaturé et éventuellement condensé à un noyau benzo, cycle qui contient au plus trois hétéroatomes pris dans l'ensemble constitué par O, N et S et qui ne porte pas de substituant ou porte un alkyle en $C_1$-$C_4$ ou un halogène, et qui peut contenir un radical carbonyle,
$R^5$ représente H, un alkyle en $C_1$-$C_6$ ou un phényle, ou encore, dans le cas où X représente un radical $-CS-OR^5$, le symbole $R^5$ représente un cation utilisable en agriculture,
les $R^6$ représentent chacun, indépendamment les uns des autres, l'hydrogène, un alkyle en $C_1$-$C_4$ ou un benzyle,
les $R^7$ représentent chacun, indépendamment les uns des autres, l'hydrogène, un alkyle en $C_1$-$C_{12}$ non substitué ou porteur d'un phényle - lui-même éventuellement porteur d'un halogène, d'un nitro, d'un cyano, d'un alkyle en $C_1$-$C_4$ ou d'un alcoxy en $C_1$-$C_4$ -, d'un hydroxy, d'un cyano, d'un ($C_1$-$C_4$-alcoxy)-carbonyle, d'un alkylthio en $C_1$-$C_4$ d'un alcoxy en $C_1$-$C_4$, d'un cycloalkyle en $C_5$-$C_7$ ou d'un benzyloxy, un alcényle en $C_3$-$C_6$ , un halogéno-alcényle en $C_3$-$C_6$, un alcynyle en $C_3$-$C_6$ , un cycloalkyle en $C_5$-$C_8$, un cyclo-alcényle en $C_5$ ou $C_6$, un ($C_1$-$C_6$-alkyl)-carbonyle, un halogéno-($C_1$-$C_6$-alkyl)-carbonyle, un [($C_1$-$C_6$-alkyl)-amino]-carbonyle, un benzoyle, un halogénobenzoyle ou un méthylbenzoyle,
les $R^8$ représentent chacun, indépendamment les uns des autres, un alkyle en $C_1$-$C_6$ qui ne porte pas de substituant ou qui porte un phényle, un cycloalkyle en $C_5$-$C_7$, un alcoxy en $C_1$-$C_4$, un alkylthio en $C_1$-$C_4$ ou un halogène, ou deux radicaux $R^8$ forment ensemble, avec les Z et l'atome de carbone auxquels ils sont liés, un hétérocycle saturé à 5 ou 6 maillons, dépourvu de substituant ou porteur d'un alkyle en $C_1$-$C_4$, d'un hydroxyalkyle en $C_1$-$C_4$, d'un halogéno-alkyle en $C_1$-$C_4$ ou d'un phényle,
les $R^9$ représentent chacun, indépendamment les uns des autres, l'hydrogène, un halogène, un alkyle en $C_1$-$C_4$, un nitro ou un cyano,
les $R^{10}$ représentent chacun, indépendamment les uns des autres, l'hydrogène, un alkyle $C_1$-$C_6$ dépourvu de substituant ou porteur d'un alcoxy en $C_1$-$C_4$, d'un triazolyle ou d'un imidazolyle, un cycloalkyle en $C_3$-$C_6$ un alcényle en $C_3$-$C_6$, un phényle ou un benzyle, ou encore les deux radicaux $R^{10}$ forment ensemble et avec l'atome de carbone auquel ils sont liés un cycloalkyle en $C_5$-$C_7$ non substitué ou porteur d'un méthyle ou d'un halogène,
$R^{11}$ représente un alkyle en $C_1$-$C_4$, un phényle, un ($C_1$-$C_6$-alkyl)-carbonyle, un benzyle, un benzoyle, un halogénobenzyle, un halogénobenzoyle ou un méthylbenzoyle,
$R^{12}$ représente l'hydrogène, un alkyle en $C_1$-$C_4$, un formyle; un ($C_1$-$C_6$-alkyl)-carbonyle, un benzoyle, un halogénobenzoyle, un méthylbenzoyle ou un trihalogénoacétyle, $R^{13}$ représente un alkyle en $C_1$-$C_4$, un phényle ou un méthylphényle,
m est égal à 0 ou à 1,
n désigne un nombre entier de 0 à 5
p désigne un nombre entier de 0 à 4 et
q désigne un nombre entier de 0 à 6, procédé caractérisé en ce qu'on fait réagir un composé répondant à la formule II :

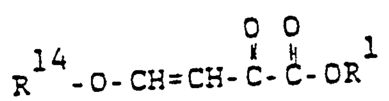
(II),

dans laquelle $R^{14}$ représente un alkyle en $C_1$-$C_6$, avec un composé de formule III :

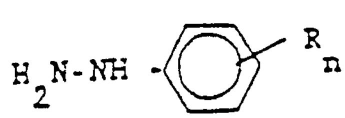
(III),

puis on transforme éventuellement le composé obtenu en l'un de ses dérivés.

**2.** Produits phytosanitaires caractérisés en ce qu'ils contiennent une quantité efficace d'un composé de formule I selon la revendication 1.

**3.** Produits régulateurs de croissance pour plantes, caractérisés en ce qu'ils contiennent une quantité efficace d'un composé de formule I selon la revendication 1.

**4.** Produits pour protéger des plantes de culture contre des actions secondaires phytotoxiques d'herbicides, produits caractérisés en ce qu'ils contiennent une quantité efficace d'un composé de formule I selon la revendication 1.

**5.** Application des composés de formule I selon la revendication 1 à la régulation de la croissance de plantes.

**6.** Application des composés de formule I selon la revendication 1 à la protection de plantes de culture contre des actions secondaires phytotoxiques d'herbicides.

**7.** Procédé pour régler la croissance de plantes, procédé caractérisé en ce qu'on applique sur les plantes ou sur la surface de culture une quantité efficace d'un composé de formule I selon la revendication 1.

**8.** Procédé pour protéger des plantes de culture contre des actions secondaires phytotoxiques d'herbicides, procédé caractérisé en ce qu'on applique sur les plantes de culture ou sur la surface de culture une quantité efficace d'un composé de formule I selon la revendication 1, avant, après ou en même temps que 1'herbicide.

**9.** Procédé selon la revendication 8, caractérisé en ce que l'herbicide est un ester d'acide phénoxyphénoxy-carboxylique ou d'acide hétéroaryloxy-phénoxycarboxylique.